# EUROPEAN PATENT SPECIFICATION

(11) **EP 2 196 537 B1**
(45) Date of publication and mention of the grant of the patent: **19.06.2013**
(21) Application number: 08836354.4
(22) Date of filing: 01.10.2008
(51) Int. Cl.: C12N 15/09, C12Q 1/68

(54) **METHOD FOR SYNTHESIS OF SINGLE- OR DOUBLE-STRANDED DNA, AND KIT FOR THE SYNTHESIS**
SYNTHESEVERFAHREN FÜR EINZEL- ODER DOPPELSTRÄNGIGE DNA UND KIT FÜR DIE SYNTHESE
PROCÉDÉ DE SYNTHÈSE D'UN ADN SIMPLE BRIN OU DOUBLE BRIN ET COFFRET DE SYNTHÈSE

(30) Priority: 01.10.2007 JP 2007258089
(43) Date of publication of application: 16.06.2010
(73) Proprietor: Wako Pure Chemical Industries, Ltd., Osaka-shi Osaka 540-8605 (JP)
(72) Inventor: HAYASHIDA, Yukinobu, Amagasaki-shi Hyogo 661-0963 (JP)
(74) Representative: von Kreisler Selting Werner
(86) International application number: PCT/JP2008/067856
(87) International publication number: WO 2009/044782

(56) References cited:
- US-A1- 2007 117 121
- HAYASHIDA YUKINOBU ET AL: "A useful approach to total analysis of RISC-associated RNA." BMC RESEARCH NOTES 2009 LNKD- PUBMED:19706194, vol. 2, 2009, page 169, XP002599456 ISSN: 1756-0500
- ARAVIN M. ET AL: 'Identification and characterization of small RNAs involved in RNA silencing' FEBS LETT. vol. 579, 2005, pages 5830 - 5840, XP005126047
- SEIKAGAKU JITEN: 'RNA', 1998, KABUSHIKI KAISHA TOKYO KAGAKU DOJIN page 86
- BLONDAL T. ET AL: 'Isolation and characterization of a thermostable RNA ligase 1 from a Thermus scotoductus bacteriophage TS2126 with good single stranded DNA ligation properties' NUCLEIC ACIDS RES. vol. 33, no. 1, 2005, pages 135 - 142, XP003026551
- YUKINOBU HAYASHIDA: 'microRNA Cloning Kit Wako no Kaihatsu to sono Oyo' WAKO JUN'YAKU JIHO vol. 76, no. 1, 15 January 2008, page 13
- FU H. ET AL: 'Identification of human fetal liver miRNAs by a novel method' FEBS LETT. vol. 579, no. 17, 2005, pages 3849 - 3854, XP004961982

## Description

### [Technical Field]

The present invention relates to a method for synthesis of single-stranded or double-stranded DNA from RNA and a kit for the synthesis.

### [Background Art]

MicroRNA comprises 18 to 25 nucleotides and is considered to be involved in various mechanisms of the eukaryotes. Particularly, in recent years, researches on embryology have been carried out actively, and several hundreds of the microRNAs have been identified in many species of the eukaryote.

Although the Neilson method is currently employed in general as a method for cloning and identifying the microRNA, the aforementioned method had problems such that (1) operation is complicated because extraction of RNA from denatured acrylamide gel has to be carried out several times; (2) the risk of RNA degradation during operation is high because the method has many steps of RNA treatment; (3) radioisotope (RI) with high risk has to be used ; and (4) use of expensive equipment such as imaging analyzer is required. Therefore, development of a method for cloning and identifying unknown RNA such as the microRNA having less than 100 nucleotides in entire length has been desired.

On the other hand, when the cloning is to be performed for RNA of which a part of sequence is known, it is common to use RACE (rapid amplification of cDNA ends) after obtaining cDNA corresponding thereto. The 3' RACE can be performed by the method hardly different from normal reverse transcription-PCR, however, in 5' RACE, the problem is that there are many steps to be performed and the operation is complicated. For example, the Oligo-Capping method, the Gene Trapper method, etc. are known as 5' RACE, however, these methods also have problem such as decrease in production efficiency of target gene caused by existence of artifact, in addition to aforementioned problems concerning number of step and operability. Therefore, development of a new 5' RACE method in which such problems have been solved, in other words, development of a new cloning method for known RNA and a new method for determining 5'-terminal has been desired.

### [Disclosure of the Invention]

### [Problem to be Solved by the Invention]

An object of the present invention is to provide a simple and safe method for synthesis of single-stranded or double-stranded DNA and a kit for performing the synthesis.

### [Means for Solving the Problem]

In view of the above-described situation, the present inventors have studied intensively on the method for cloning unknown RNA which do not have problems as described above but can also perform the cloning of the RNA having less than 100 nucleotides like microRNA, as well as the method for cloning RNA having in part of known nucleotide sequence which do not have problems as described above. Consequently they found that, in the synthetic reaction of DNA corresponding to template RNA, impurities such as unreacted RNA and RNA derivatives can be removed easily by conducting alkaline treatment after the reverse transcription reaction, and the objective DNA can be obtained easily in consequence, and further found that by employing aforementioned method, it is not necessary to extract RNA from acrylamide gel nor to use special equipment such as imaging analyzer. In addition, they have also found that according to the aforementioned method, impurities such as unreacted RNA and RNA derivatives can be removed easily, and artifact can be reduced accordingly, and therefore the DNA comprising objective sequence corresponding to the template RNA can be amplified efficiently by PCR, and thus completed the present invention.

The present disclosure relates to the followings:
(1) a method for synthesis of a single-stranded DNA containing a nucleotide sequence corresponding to the template RNA, characterized by comprising the following steps:
   1) Step 1 in which the template RNA is subjected to reverse transcription reaction;
   2) Step 2 in which the solution processed in Step 1 is subjected to alkaline treatment;
(2) a method for synthesis of a single-stranded DNA containing a nucleotide sequence corresponding to the template RNA, characterized by comprising the following steps:
   1) a step in which an RNA fragment of known sequence is added to 3'-terminal of the template RNA;
   2) Step 1 in which the template RNA is subjected to reverse transcription reaction;
   3) Step 2 in which the solution processed in Step 1 is subjected to alkaline treatment;
(3) a method for synthesis of a double-stranded DNA containing a nucleotide sequence corresponding to the template RNA, characterized by comprising the following steps:
   1) Step 1 in which the template RNA is subjected to reverse transcription reaction;
   2) Step 2 in which the solution processed in Step 1 is subjected to alkaline treatment;
   3) Step 3 in which the obtained single-stranded DNA is converted to a double-stranded;
(4) a method for synthesis of a double-stranded DNA containing the nucleotide sequence corresponding to the template RNA, characterized by comprising the following steps:
   1) a step in which an RNA fragment of known sequence is added to 3'-terminal of the template RNA;
   2) Step 1 in which the template RNA is subjected to reverse transcription reaction;
   3) Step 2 in which the solution processed in Step 1 is subjected to alkaline treatment;
   4) Step 3 in which the obtained single-stranded DNA is converted to a double-stranded DNA;
(5) a kit for synthesis of single-stranded DNA containing a nucleotide sequence corresponding to the template RNA, comprising I) an enzyme capable of adding RNA fragment to single-stranded RNA and III) a reverse transcriptase;
(6) a kit for synthesis of double-stranded DNA containing a nucleotide sequence corresponding to the template RNA, comprising I) an enzyme capable of adding RNA fragment to single-stranded RNA, II) an RNA fragment of known sequence, III) a reverse transcriptase, IV) a primer for reverse transcription reaction, V) a mixture of deoxyribonucleotide triphosphates (dNTPs), VI) an enzyme capable of dephosphorilating 5'-terminal phosphate group of RNA, VII) a DNA fragment of known sequence, VIII) an enzyme capable of adding DNA fragment to the single-stranded DNA, IX) an enzyme capable of dephosphorilating 5'-terminal phosphate group of the DNA, and XI) a PCR primer; and
(7) a kit for synthesis of double-stranded DNA containing a nucleotide sequence corresponding to the template RNA, comprising II) an RNA fragment of known sequence, III) a reverse transcriptase, IV) a primer for reverse transcription reaction, V) dNTPs, VI) an enzyme capable of dephosphorilating 5'-terminal phosphate group of the RNA, VII) a DNA fragment of known sequence, IX) an enzyme capable of dephosphorilating 5'-terminal phosphate group of the DNA, and XI) PCR primer.

### [Effect of the Invention]

According to the present invention, syntheses of single-stranded DNA and double-stranded DNA from RNA can be performed by simple operations, in safety without use of RI, and without need of expensive apparatus such as imaging analyzer. In addition, since the impurities, such as unreacted RNA and RNA derivatives, can be removed easily, synthesis of single-stranded DNA corresponding to the objective mRNA is enabled using the total RNA; and in the synthesis of double-stranded DNA, it becomes possible to increase the PCR amplification efficiency of the target DNA. Therefore, by using the obtained DNA, cloning of DNA is also made possible simply and safely without employing expensive equipment. Furthermore, the method of the present invention is also possible to be applied to various RNA because of not depending on the CAP-structure of the RNA.

### [Brief Description of Drawings]

[Fig. 1]

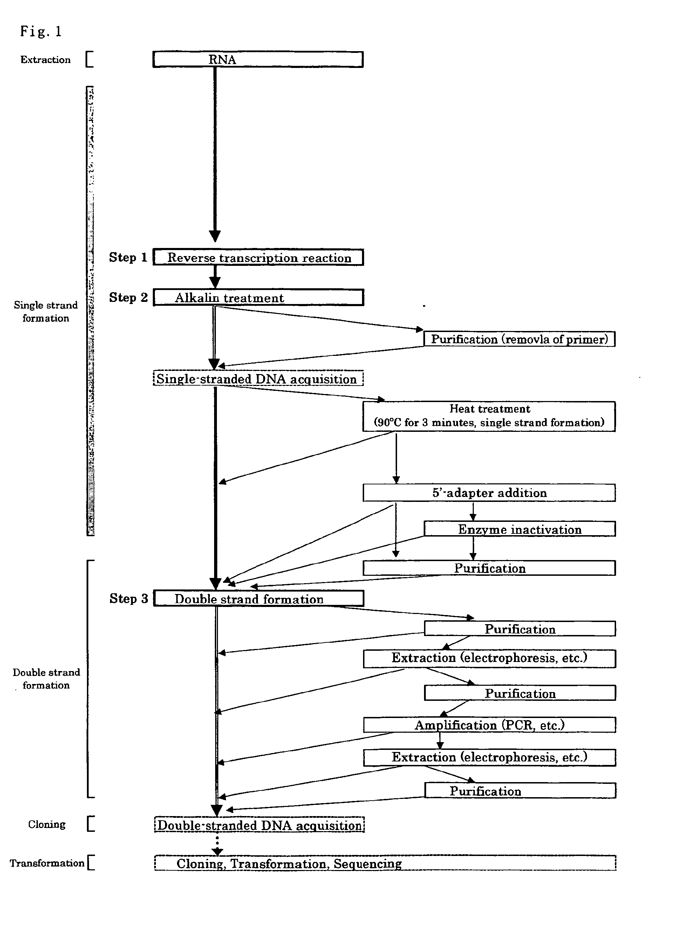

   Fig. 1 shows main schematics of the synthesis method of the present disclosure. The "Single-stranded DNA acquisition" and the "Double-stranded DNA acquisition" do not represent steps but represent the obtained results, and "cloning, transformation, and sequencing" represent the nucleotide sequence analysis of the obtained double-stranded DNA.
[Fig. 2]

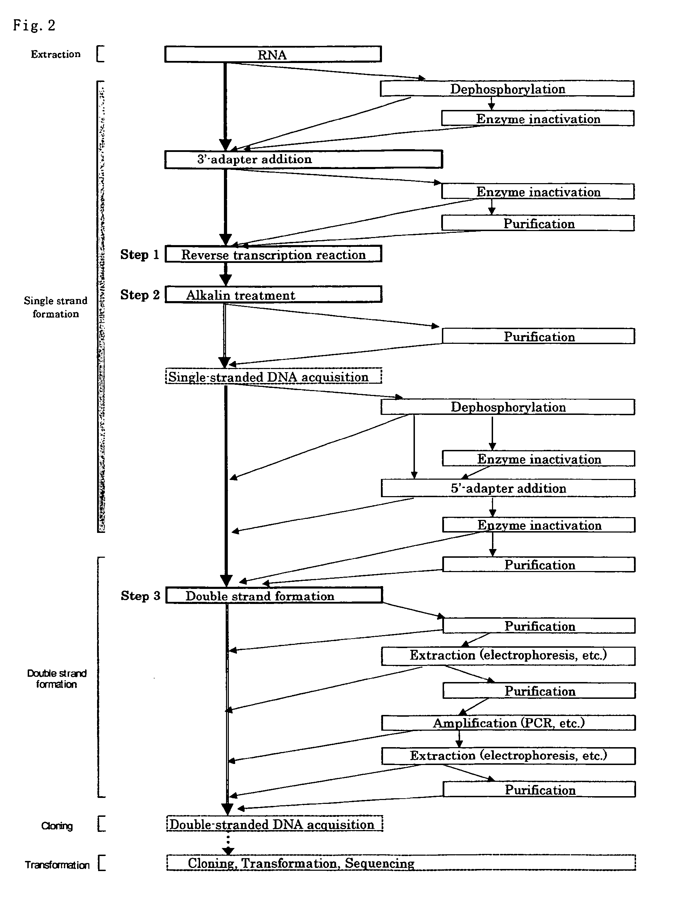

   Fig. 2 shows main schematics of the synthesis method of the present disclosure including 3'-adapter addition step.
[Fig. 3]

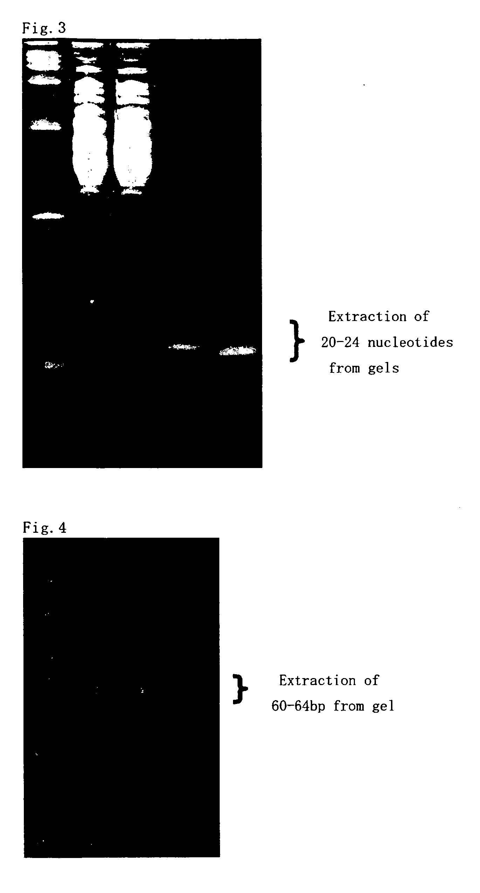

   Fig. 3 shows an image of the gel after electrophoresis which was performed by introducing small RNA fraction other than 200 nucleotides obtained in the 1st section of Example 1 into denatured polyacrylamide gel.
[Fig. 4]
   Fig. 4 shows an image of the gel after electrophoresis of the cDNA for cloning obtained by the PCR in the 11th section of Example 1.
[Fig. 5]

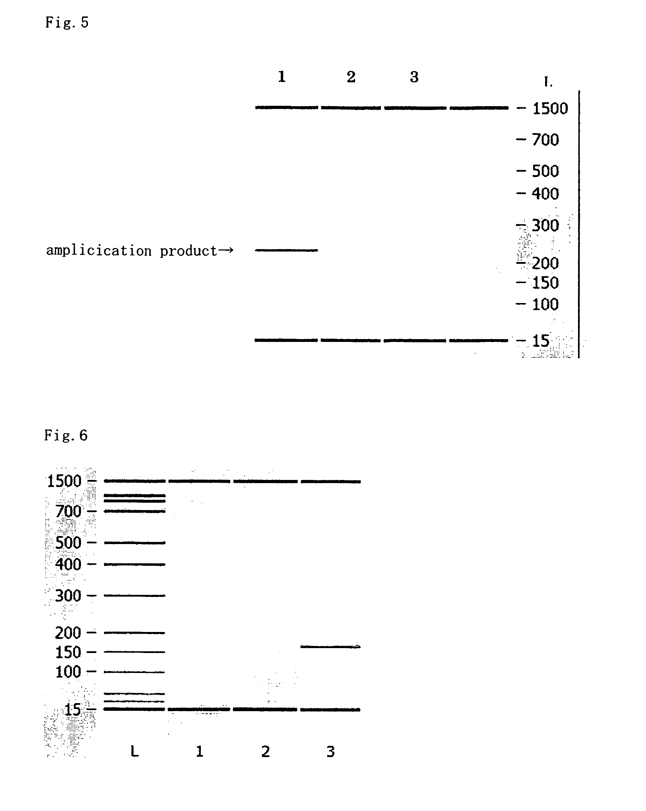

   Fig. 5 shows an image of the gel after electrophoresis of the cDNA for cloning obtained by the PCR in the 6th section of Example 2.
[Fig. 6]
   Fig. 6 shows an image of the gel after electrophoresis of the cDNA for cloning obtained by the PCR in the 6th item of Example 3. Lane 1 shows the result of amplification using only the PCR primer which is complementary to the adapter; Lane 2 shows the result of amplification using only the PCR primer which is complementary to p16 INK4a mRNA; and Lane 3 shows the result of amplification using the PCR primer which is complementary to the adapter and the PCR primer which is complementary to p16 INK4a mRNA.

### [Best Mode for Carrying Out the Invention]

In the method for synthesis of single-stranded DNA or double-stranded DNA of the present invention (hereinafter, both methods are optionally briefly termed as the synthesis methods of the present invention), the template RNA is not particularly limited, so long as it is the RNA commonly employed in this field, and specifically includes, for example, ribosomal RNA, messenger RNA, transfer RNA, small nucleic acid RNA (small nuclear RNA), small RNA, microRNA and the like. When RNA of unknown nucleotide sequence (hereinafter, optionally briefly termed as an unknown RNA) is employed as a template RNA, messenger RNA, small RNA, microRNA and the like are preferable; among them, small RNA and microRNA with nucleotide chain having 100 or less nucleotides are more preferable, and microRNA is particularly preferable. When RNA of which a part of the nucleotide sequence is known (hereinafter, optionally briefly termed as a known RNA) is employed as a template RNA, messenger RNA is preferable. It should be noted that, the aforementioned known RNA represents RNA which has a known sequence of at least 20 or more, preferably 40 or more of nucleotides and the 5'-terminal of which is unknown, preferably RNA which has further unknown sequence of 30 or more of nucleotides at the 5' side of the known sequence.

### Method for synthesis of single-stranded DNA

The method for synthesis of single-stranded DNA containing a nucleotide sequence corresponding to the template RNA of the present disclosure (hereinafter, optionally briefly termed as the synthesis method of single-stranded DNA of the present invention) is characterized by comprising the following steps:
(1) Step 1 in which the template RNA is subjected to reverse transcription reaction;
(2) Step 2 in which the solution processed in the Step 1 is subjected to alkaline treatment.

In the method for synthesis of single-stranded DNA of the present invention, the single-stranded DNA represents the one which contains a nucleotide sequence corresponding to the template RNA, and the one which contains a part of nucleotide sequence corresponding to the template RNA. The single-stranded DNA which contains a part of nucleotide sequence corresponding to the aforementioned template RNA includes, for example, a single-stranded DNA obtained by subjecting the aforementioned RNA to the reverse transcription reaction using a primer for the reverse transcription reaction, which is capable of annealing to the known sequence in the known RNA, wherein the template RNA is known RNA. Specifically, for example, a single-stranded DNA which contains a nucleotide sequence corresponding to the sequence from the known sequence to the 5'-terminal in the template RNA, and the like are included.

The amount of the template RNA to be used in the above-described Step 1 is not particularly limited because it changes depending on the sample employed, but it is usually lng to 50µg as an amount of nucleic acid. For example, when total RNA is used as a sample, the sample is usually lng to 50µg, preferably 1 to 20µg; however, when an RNA separated as a fraction by electrophoresis is used, it is usually 1 to 100 ng. It should be noted that, these RNA is usually subjected to the aforementioned step in a solution containing the above described amount of nucleic acid, and the amount in solution is usually 1 to 30 µL, preferably 1 to 20 µL. In addition, as for a solvent of the sample solution containing RNA, usually, sterilized distilled water is used.

The above-described Step 1 may be carried out by subjecting the template RNA to the reverse transcription reaction according to the method well known per se and usually used in this field, and also can be carried out using a commercially available kit. It may be carried out, for example, according to the method described in Nucleic Acids Research, 1988, Vol. 16, No. 5, 1999-2014 or Nucleic Acids Research, 1988, Vol. 16, No. 1, 265-277. Specifically, for example, to the template RNA, a reverse transcriptase, a primer for reverse transcription reaction, a mixture of four kinds of deoxyribonucleotide triphosphates (dNTPs) are added, and reacted in a buffer solution such as Tris-HCl buffer (pH 8.3), imidazole (pH 8.2) and the like at usually 35 to 50°C, preferably 40 to 50°C for usually 30 to 90 minutes, preferably 30 to 60 minutes. After that the reaction is terminated by heat-treatment or addition of reaction-terminating solution. Thereby, the single-stranded DNA which is complementary to the template RNA can be obtained.

The reverse transcriptase to be used in the above-described reverse transcription reaction is not particularly limited so long as the enzyme is used conventionally in this field. The transcriptase includes, for example, Moloney Murine Leukemia Virus (M-MLV) reverse transcriptase, Avian myeloblastosis virus (AMV) reverse transcriptase, M-MLV reverse transcriptase (RNase H negative) and the like. Among them, M-MLV reverse transcriptase (RNase H negative) is preferable. In addition, although amount thereof to be used varies depending on type of the enzyme employed, it is usually 1 to 100 units, preferably 10 to 200 units for 1 µg of nucleic acid of the template RNA.

The primer for reverse transcription reaction may be the one which is capable of annealing to the template RNA, and serves as a starting point of DNA chain extension. When the template RNA is known RNA, the one which is capable of annealing to the known sequence or to a part of the known sequence is preferable. Number of nucleotides of the primer for reverse transcription reaction is usually 10 to 50, preferably 12 to 40, and more preferably 15 to 30. Amount of the primer to be used is usually 1 to 250 pmol, preferably 10 to 50 pmol per 1 µg of nucleic acid of the template RNA. The above-described dNTPs are a mixture of four kinds of deoxyribonucleotide triphosphates usually employed in this field, and amount thereof to be employed is usually 0.1 to 20 nmol, preferably 1 to 10 nmol per 1 µg of nucleic acid of the template RNA. The above-described reaction terminating solution includes, for example, EDTA and the like, and amount thereof to be used is usually 10 to 100 mmol/L, and preferably 40 to 60 mmol/L as a final concentration. In addition, the heat treatment for stopping the reaction is carried out usually at 65 to 100°C, preferably at 65 to 70°C, and usually for 15 to 60 minutes, preferably for 15 to 30 minutes. In the above-described reverse transcription reaction, the reagent usually used for such reverse transcription reaction in this field, for example, reducing agents such as DTT (dithiothreitol), potassium chloride, magnesium chloride, and ribonuclease inhibitor may be added. Concentration and amount of these reagents to be used are set appropriately from the ranges usually employed in this field.

The above-described Step 2 may be carried out by subjecting the solution processed in Step 1, namely the solution comprising a single-stranded DNA which is complementary to the template RNA, to alkaline treatment. By the aforementioned step, remaining RNA such as residual template RNA can be decomposed.

The above-described alkaline treatment may be any method which is capable of decomposing RNA, and is performed, for example, by making the reaction solution alkaline by adding alkali or aqueous solution thereof to the reaction solution. The aforementioned alkali includes, for example, alkali metal hydroxides such as sodium hydroxide, potassium hydroxide and the like, alkaline earth metal hydroxide such as barium hydroxide, magnesium hydroxide, calcium hydroxide and the like, alkali metal carbonate such as sodium carbonate, ammonia, and amine. Among them, alkali metal hydroxides such as sodium hydroxide, potassium hydroxide and the like are preferable, and the like, and above all, sodium hydroxide is particularly preferable. Specifically, the aforementioned alkaline treatment is carried out by adjusting pH of the aforementioned solution to 10 to 14, preferably 12 to 14, and heating usually at 50 to 80°C, preferably at 60 to 70 °C, usually for 20 to 120 minutes, preferably for 20 to 60 minutes.

In the above-described Step 2, more preferably, the single-stranded DNA which is complementary to the template RNA is purified after the alkaline treatment by the method conventionally used in this field, such as, for example, extraction with a mixed solution of phenol/chloroform/isoamyl alcohol, alcohol precipitation, column refining, and filtration through a filter.

In the above-described method for synthesis of single-stranded DNA of the present invention, it is preferable to use the known RNA as a template RNA. As a preferable example of the aforementioned synthesis method, the specific example when known RNA is used as a template RNA is explained below.

That is, first, to a solution (e.g. 10 mL) which comprises 1 to 20 ng (as nucleic acid amount) of template RNA having a known sequence, 1 to 2 µL of a solution which comprises 10 to 50 pmol/L of a primer which is Complementary to the known sequence in the template RNA and 1 to 2 µL of a solution which comprises 100 to 300 units/µL of a reverse transcriptase are added. The mixture is allowed to react at 40 to 50°C for 30 to 60 minutes, and thereby a single-stranded DNA which is complementary to the template RNA can be obtained. Further, to a solution comprising a single-stranded DNA which is complementary to the template RNA, for example, hydroxide of alkali metal such as sodium hydroxide is added so as to make pH of the reaction solution to be 12 to 14. By incubating this solution at 60 to 70°C for 30 to 60 minutes, the residual RNA can be decomposed. Subsequently, by refining the obtained single-stranded DNA by purifying using a method such as extraction, for example, with a mixed solution of phenol/chloroform/isoamyl alcohol, etc., the single-stranded DNA involved in the present invention which contains a nucleotide sequence complementary to the template RNA can be obtained.

### • Method for synthesis of single-stranded DNA including 3'-adapter addition step

The method for synthesis of single-stranded DNA of the present invention may further includes, prior to the above-described Step 1, a method which includes the step where an RNA fragment of known sequence is added to 3'-terminal of the template RNA. Specifically, the method is carried out by the following steps:
(1) a step in which an RNA fragment of known sequence (hereinafter, optionally briefly termed as 3'-adapter involved in the present invention) is added to 3'-terminal of the template RNA,
(2) Step 1 in which the template RNA added with the above-described RNA fragment (hereinafter, optionally briefly termed as 3'-adapter-bound RNA) is subjected to the reverse transcription reaction,
(3) Step 2 in which the solution processed in the Step 1 is subjected to alkaline treatment.

In this case, 3'-adapter involved in the present invention may have any sequence, so long as it has been treated so that hydroxyl group of 3'-side does not react with phosphate group, and the sequence is a known RNA. The one which consists of a sequence not existing in the template RNA nor in the DNA complementary thereto is preferable. The treatment by which the hydroxyl group of 3'-side does not react with a phosphate group may be a method usually performed in this field, and includes, for example, a treatment such as dehydroxylation of the hydroxyl group of 3'-terminal, binding of biotin to the hydroxyl group of 3'-terminal, and the like. Among them, the treatment of dehydroxylation of 3'-terminal hydroxyl group is preferable. Number of nucleotides of the 3'-adapter involved in the present invention is usually 12 to 45, preferably 15 to 30, and more preferably 17 to 25.

As to the step in which 3'-adapter involved in the present invention is added to the 3'-terminal of the above-described template RNA (hereinafter, optionally briefly termed as 3'-adapter addition step), 3'-adapter involved in the present invention may be added to the 3'-terminal of the template RNA by a method well known per se and usually used in this field, and also can be carried out using a commercially available kit. It may be carried out, for example, according to the method described in Nucleic Acids Research, 1984, Vol. 12, No. 21, 8235-8251 or Nucleic Acids Research, 1991, Vol. 19, No. 19, 5227-5232. Specifically, it can be carried out in the presence of an enzyme which is capable of binding a single-stranded RNA and a single-stranded RNA (hereinafter, optionally briefly termed as the single-stranded RNA ligase involved in the present invention), by incubating the template RNA and the 3'-adapter involved in the present invention in a buffer solution such as HEPES, MOPS at usually 50 to 70°C, preferably 60 to 70°C for 30 to 90 minutes, preferably 30 to 60 minutes. Thereby, 3'-adapter-bound RNA can be obtained.

Amount of the template RNA employed in the 3'-adapter addition step is same as described in the paragraph of method for synthesis of single-stranded DNA.

The single-stranded RNA ligase involved in the present invention employed herein is not particularly limited so long as it is an enzyme which is capable of binding a single-stranded RNA and a single-stranded RNA, including also an enzyme which is capable of binding a single-stranded RNA and a single-stranded RNA as well as a single-stranded DNA and a single-stranded DNA. Specifically, for example, RNA ligase such as T4 RNA ligase, DNA ligase such as thermostable single-stranded DNA ligase as described in the literature such as Nucleic Acids Research, 2005, Vol. 33, No. 1 135-142, etc. are included, and among them, the thermostable single-stranded DNA ligase and the like in the above-described literature is preferable. Amount of the single-stranded RNA ligase involved in the present invention to be used in the above-described reaction may vary depending on the kind of ligase employed, however, it is usually 1 to 50 units, preferably 5 to 15 units for 1 µg of nucleic acid of the template RNA.

Amount of the 3'-adapter involved in the present invention to be used is 10 to 200 pmol, preferably 50 to 100 pmol for 1 µg of nucleic acid of the template RNA.

In the above-described reaction, reagents which are usually employed in such ligation reaction, for example, coenzymes such as ATP (adenosine triphosphate), reducing agents such as DTT (dithiothreitol), magnesium chloride, BSA, and manganese chloride may be added, and concentration and amount of these reagents to be employed are set appropriately from the range usually used in this field.

In this regard, in the above-described 3'-adapter addition step, to prevent self-binding of the template RNA, it is preferable to treat the template RNA in advance so that phosphate group in the 5'-terminal side does not react with hydroxyl group of the template RNA. The treatment may be carried out according to the method usually performed in this field, for example, dephosphorylation treatment by dephosphorylating enzyme and the like are included. The dephosphorylating enzyme to be used for the dephosphorylation treatment includes, for example, SAP (Shrimp Alkaline Phosphatase), BAP (Bacterial Alkaline Phosphatase), CIAP (Calf Intestine Alkaline Phosphatase), etc.;and, SAP (Shrimp Alkaline Phosphatase) which is easy to conduct enzyme-inactivation treatment is preferable. The dephosphorylation treatment by dephosphorylating enzyme is performed, for example, by adding the dephosphorylating enzyme with usually 0.1 to 10 units, preferably 0.5 to 5 units for 1 µg of nucleic acid of the template RNA, and allowing the mixture to react in an appropriate buffer solution such as Tris-HCl, HEPES, MOPS, etc., usually at 30 to 45°C, preferably at 35 to 40 °C, and usually for 15 to 60 minutes, preferably for 15 to 30 minutes. In the case where dephosphorylation treatment is carried out using dephosphorylating enzyme, it is preferable to inactivate or to remove the dephosphorylating enzyme after the treatment by a method well-known per se. For example, in the case where SAP is used, heating is preferable as an inactivation method, usually at 65 to 100°C, preferably at 65 to 90°C, more preferably at 65 to 80°C, and usually for 15 to 90 minutes, preferably for 15 to 60 minutes.

In the above-described 3'-adapter addition step, after the step, it is preferable to inactivate the applied single-stranded RNA ligase involved in the present invention. The inactivation process may be performed according to a method well known per se depending on the enzyme employed, for example, by heat treatment usually at 80 to 95°C, preferably at 80 to 90°C, and usually for 5 to 15 minutes, preferably for 5 to 10 minutes. In addition, after the 3'-adapter addition step, it is more preferable to refine the obtained 3'-adapter-bound RNA by a purification method usually used in this field such as, for example, extraction by a mixed solution of phenol/chloroform/isoamyl alcohol, alcohol precipitation, column purification, filtration through filter and the like.

Specific methods of Step 1 and Step 2, reagents and amounts thereof to be used, etc. in the method for synthesis of single-stranded DNA of the present invention including 3'-adapter addition step are same as those described in the paragraph of the above-described method for synthesis of single-stranded DNA (method for synthesis of single-stranded DNA which does not include 3'-adapter addition step) except for the primer for reverse transcription reaction. As to the primer for reverse transcription reaction, the one which is capable of annealing to the 3'-adapter involved in the present invention and just serve as a starting point of DNA chain extension is preferable and the one which comprises a sequence complementary to the 3'-adapter are more preferable. Among them, the one which does not comprise a sequence complementary to the template RNA is particularly preferable. Number of nucleotides of the primer for reverse transcription reaction, amount of the primer to be used, and method for using it are same as those described in the paragraph of the above-described method for synthesis of single-stranded DNA of the present invention.

The method for synthesis of single-stranded DNA including 3'-adapter addition step of the present disclosure is preferable carried out by the following steps:
1) a step in which the 5'-terminal of template RNA is dephosphorylated;
2) a step in which an RNA fragment of known sequence is added to the 3'-terminal of template RNA;
3) a step in which the above-described RNA fragment-added template RNA is subjected to the reverse transcription reaction;
4) a step in which the solution processed in the Step 2 is subjected to alkaline treatment.

In the above-described method for synthesis of single-stranded DNA of the present disclosure including 3'-adapter addition step, it is preferable to employ unknown RNA as a template RNA having no consensus sequence such as Poly(A)⁺ sequence which exists in mRNA and the like. As a preferred example of the aforementioned method for synthesis of single-stranded DNA, specific method when unknown RNA is employed is explained below.

That is, first, a solution (e.g. 10 µL) which contains 1 ng to 1 µg (as amount of nucleic acid) of a template RNA is incubated with 0.5 to 3 µL of a solution containing 0.5 to 3 units/L dephosphorylating enzyme such as, for example, SAP, for example, at 30 to 40°C for15 to 30 minutes, and thereby 5'-terminal of the template RNA is dephosphorylated. After that, the dephosphorylating enzyme such as SAP is inactivated by incubation at 60 to 70°C for 15 to 30 minutes. Then, to the solution containing 5'-terminal-dephosphorylated template RNA, 1 to 2 µL of a solution containing 5 to 15 units/L of a single-stranded RNA ligase involved in the present disclosure, and 1 to 2 µL of a solution containing 10 to 100 µmol/L of 3'-terminal-dehydroxylated 3'-adapter involved in the present disclosure are added and incubated at 60 to 70°C for 30 to 60 minutes, thus the 3'-adapter-bound RNA is obtained. After that, the aforementioned single-stranded RNA ligase is inactivated by heating at 90 to 100°C for 5 to 10 minutes. Subsequently, the 3'-adapter-bound RNA is refined, for example, by a refining method using phenol/chloroform/isoamyl alcohol, etc. Then, to the solution (e.g. 10 µL) which contains 3'-adapter-bound RNA, 1 to 2 µL of a solution containing 10 to 100 µmol/L of a primer which is complementary to 3'-adapter and 1 to 2 µL of a solution containing 100 to 200 units/L of a reverse transcriptase are added, and incubated at 40 to 50°C for 30 to 60 minutes, thus the single-stranded DNA which is complementary to the 3'-adapter-bound RNA is obtained. Further, to the solution containing the single-stranded DNA which is complementary to the 3'-adapter-bound RNA, for example, hydroxide of alkali metal such as sodium hydroxide is added so as to make pH of the reaction solution 12 to 14, and by incubation at 60 to 70°C for 30 to 60 minutes, the residual RNA can be decomposed. Then, by refining the obtained single-stranded DNA, for example, by a refining method employing phenol/chloroform/isoamyl alcohol, etc., the single-stranded DNA involved in the present invention which comprises nucleotide sequence corresponding to the template RNA can be obtained.

According to the above-described method for synthesis of single-stranded DNA of the present disclosure, the single-stranded DNA which comprises nucleotide sequence corresponding to various types of template RNA (hereinafter, optionally briefly termed as the single-stranded DNA involved in the present invention) can be obtained safely and easily.

### • Method for synthesis of double-stranded DNA

The method for synthesis of double-stranded DNA which contains a nucleotide sequence corresponding to the template RNA involved in the present disclosure (hereinafter, optionally briefly termed as the synthetic method of double-stranded DNA of the present invention) is characterized by comprising the following steps:
1) Step 1 in which the template RNA is subjected to reverse transcription reaction;
2) Step 2 in which the solution processed in the Step 1 is subjected to alkaline treatment;
3) Step 3 in which the obtained single-stranded DNA is converted to a double-stranded DNA.

The above-described Step 1 and Step 2 are the same steps as Steps 1 and 2 in the above-described paragraph of method for synthesis of single-stranded DNA of the present disclosure, and preferred procedures are also the same. That is, the double-stranded DNA which comprises nucleotide sequence corresponding to the template RNA can be synthesized by subjecting the single-stranded DNA involved in the present disclosure obtained by passing through the above-described Steps 1 and 2, to Step 3.

In Step 3, the single-stranded DNA involved in the present invention obtained in the Step 2 of the above-described method for synthesis of single-stranded DNA of the present disclosure may be converted to a double-stranded DNA using a method usually employed in this field or a commercially available kit. The method for forming a double-stranded DNA using Klenow fragment as described in Proceedings of the National Academy of Sciences USA, 1997, Vol. 74, No. 2, 5463-5467, and Anal. Biochem., 1983, Vol.132, 6-13, or the method for converting a single strand to a double-stranded DNA by the PCR as described in Nucleic Acids Research, 1991, Vol.19, 3749, and BioTechniques, 1994, Vol.16, 1134-1137 are included. Among them, the method by PCR is particularly preferable because it is suitable for cloning. The method for converting a single-stranded DNA to a double-stranded DNA by PCR is carried out, for example as follows:

That is, first, for example, by using an enzyme which is capable of binding a single-stranded DNA and a single-stranded DNA (hereinafter, optionally briefly termed as the single-stranded DNA ligase involved in the present invention), a known DNA fragment (hereinafter, briefly termed as the 5'-adapter involved in the present disclosure) is added to 5'-terminal of the single-stranded DNA involved in the present invention obtained in the Step 2 (DNA obtained in this way is optionally briefly termed as the "5'-adapter-bound single-stranded DNA involved in the present disclosure). After that, primer 1 which is designed from the aforementioned 5'-adapter and primer 2 which is designed from complementary strand of the primer for reverse transcription reaction which is capable of annealing to the known sequence of RNA are prepared. Then, using these primer 1 and primer 2, PCR reaction is carried out for the single-stranded DNA involved in the present invention added with 5'-adapter involved in the present disclosure. In this connection, the step in which the 5'-terminal of the single-stranded DNA involved in the present disclosure obtained in Step 2 is added with the 5'-adapter involved in the present disclosure may be included in the above-described step of the method for synthesis of single-stranded DNA which comprises the method for synthesis of single-stranded DNA and 3'-adapter addition step. That is, the method for synthesis of single-stranded DNA and the method for synthesis of single-stranded DNA including 3'-adapter addition step, which include the 5'adapter addition step in the present disclosure, also becomes one aspect of the method for synthesis of single-stranded DNA of the present invention.

The single-stranded DNA ligase involved in the present invention to be employed herein may be an enzyme which is capable of binding a single-stranded DNA and a single-stranded DNA, including an enzyme which is capable of binding a single-stranded RNA and a single-stranded RNA as well as a single-stranded DNA and a single-stranded DNA. For example, the DNA ligase such as thermostable single-stranded DNA ligase as described in the literature such as Nucleic Acids Research, 2005, Vol. 33, No. 1 135-142, is preferable. When such a thermostable single-stranded DNA ligase is utilized, adapter ligation can be conducted at a high temperature of 50 to 70°C, and as a result, the DNA and adapter can be prevented from forming secondary structure, and consequently, it becomes possible to carry out the ligation efficiently.

The 5'-adapter involved in the present invention has been processed so that hydroxyl group of 3'-side does not react with a phosphate group. Therefore, the sequence may be any type of sequence, so long as it is known DNA, but the one which consists of a sequence not existing in the single-stranded DNA involved in the present invention nor in the complementary strand thereto is preferable. Number of nucleotides thereof is usually 12 to 30, preferably 15 to 25, and more preferably 18 to 22. The treatment by which the hydroxyl group of 3'-side does not react with a phosphate group may be a method usually carried out in this field, for example, a treatment such as dehydroxylation of the hydroxyl group of 3'-terminal, and binding of biotin to the hydroxyl group of 3'-terminal are included. Among them, the treatment of dehydroxylation of 3'-terminal hydroxyl group is preferable. The one which has been dehydroxylated can be extracted easily from a mixed solution of phenol/chloroform/isoamyl alcohol, because it dissolves in an aqueous solution when the mixed solution of phenol/chloroform/isoamyl alcohol as a refining method is carried out.

The above-described primer 1 is the one which comprises the entire or a part of a complementary strand to the 5'-adapter involved in the present invention, and number of nucleotides thereof is usually 12 to 30, preferably 15 to 25, more preferably 18 to 22. The above-described primer 2 is the one which comprises the entire or a part of a complementary strand to the primer for reverse transcription reaction which is capable of annealing to the known sequence of RNA, and number of nucleotides thereof is usually 12 to 30, preferably 15 to 25, more preferably 18 to 22. As for these primers 1 and 2, the ones having a sequence which does not exist in the single-stranded DNA involved in the present invention and complementary strand thereto are preferable.

Method of adding the 5'-adapter involved in the present invention to the 5'-terminal of the single-stranded DNA involved in the present invention in the method of converting the single-stranded DNA involved in the present invention to the double-stranded DNA by PCR may be preformed by adding the 5'-adapter involved in the present invention to the 5'-terminal of target DNA by a method well known per se usually employed in this field, and also it can be performed using a commercially available kit. It may be performed according to the method described, for example in Nucleic Acids Research, 1988, Vol. 16, No. 5 1999-2014, and Nucleic Acids Research, 1988, Vol. 16, No. 1 265-27. Specifically, for example, the single-stranded DNA obtained through Steps 1 to 3 using 1 to 100 µg (as amount of nucleic acid) of template RNA is allowed to react with usually 10 to 100 pmol, preferably 10 to 50 pmol of the 5'-adapter involved in the present invention and 1 to 50 units, preferably 5 to 15 units of single-stranded DNA ligase in a buffer solution such as HEPES, Tris-HCl, MOPS and the like at usually 50 to 70°C, preferably 50 to 60°C for 30 to 90 minutes, preferably 30 to 60 minutes. Thereby, the 5'-adapter-bound single-stranded DNA involved in the present invention can be obtained. In the aforementioned reaction, reagents which are usually employed in such ligation reaction, for example, coenzymes such as ATP (adenosine triphosphate), reducing agents such as DTT (dithiothreitol), magnesium chloride, BSA, and manganese chloride may be added, and concentration and amount of these reagents to be employed are set appropriately from the range usually used in this field.

In the method of adding the 5'-adapter involved in the present invention to the 5'-terminal of the single-stranded DNA involved in the present invention, before the addition, a treatment is preferably added so that residual substances after the reactions in Step 1 and Step 2, i.e., unreacted or decomposed adapter, primer, deoxyribonucleotide triphosphate, etc. are not react with the hydroxyl group of 5'-terminal side of the aforementioned single-stranded DNA. Such treatment may be carried out according to a method usually carried out in this field, and include, for example, removal of the residual substances by refining, dephosphorylation treatment by dephosphorylating enzyme and the like. Specifically, when the above-described single-stranded DNA involved in the present invention comprises 100 or more nucleotides, the above-described residual substances are removed using filters such as silica gel membrane or a column, etc. which are usually used in this field. Also, the residual substances may be removed using a commercially available purification kit. When the single-stranded DNA involved in the present invention comprises 100 or less nucleotides, since it is difficult to remove the residual substances by the above-described filter or a column, it is performed by dephosphorylation treatment of phosphate group of the remainder. The dephosphorylating enzyme employed herein includes the same one as the dephosphorylating enzyme employed for use in the dephosphorylation treatment carried out prior to Step 1 in the description of the above-described Step 1, and preferable dephosphorylating enzymes, inactivation/removal method, etc. are also the same to the above.

In addition, when the above-described single-stranded DNA involved in the present invention comprises 100 or more nucleotides, after performing the above-described refining treatment, preferably a heat treatment is carried out usually at 80 to 100°C for 3 to 5 minutes to convert to a single strand DNA. By carrying out such treatment, DNA can be prevented from forming secondary structure and thereby 5'-adapter can be added efficiently.

After addition of the 5'-adapter involved in the present invention to the 5'-terminal of template RNA, it is preferable to make the added single-stranded DNA ligase inactive, and the method of inactivation may be performed according to a method well known per se depending on the enzyme employed, and may be performed by heat-treatment, for example, usually at 90 to 100°C, preferably 90 to 95°C for usually 5 to 15 minutes, preferably 5 to 10 minutes. After the enzyme inactivation procedure, it is preferable to refine the 5'-adapter-bound single-stranded DNA involved in the present invention by a purification method usually used in this field such as, for example, extraction by a mixed solution of phenol/chloroform/isoamyl alcohol, alcohol precipitation, column purification, filtration through filter, and the like.

Method for carrying out the PCR reaction for the 5'-adapter-bound single-stranded DNA involved in the present invention in the process of converting a single-stranded DNA involved in the present invention to a double-stranded DNA by PCR may be preformed according to a method well known per se, for example, according to the method described in Nucleic Acids Research, 1991, Vol.19, 3749, and BioTechniques, 1994, Vol.16, 1134-1137. Specifically, it is performed as follows. Namely, to the single-stranded DNA involved in the present invention obtained through Steps 1 to 3 using 1 (µg (as amount of nucleic acid) of template RNA, usually 0.1 to 100 pmol, preferably 0.1 to 50 pmol of the above-described primer 1, usually 0.1 to 100 pmol, preferably 0.1 to 50 pmol of the above-described primer 2, and usually 0.01 to 20 nmol, preferably 0.01 to 10 nmol of 4 types of deoxyribonucleotide triphosphates (dNTPs) are added, and reacted in a buffer solution such as Tris-HCl buffer, for example, by a cycle of (1) 93 to 98°C for 1 to 10 minutes → (2) 93 to 98°C for 10 to 30 seconds → 50 to 60°C for 10 to 30 seconds → 68 to 72°C for 10 to 30 minutes (10 to 20 cycles), (3) 68 to 72°C for 1 to 5 minutes, and thereby amplified double-stranded DNA can be obtained. In the above-described PCR reaction, after the reaction, it is preferable to refine the obtained double-stranded DNA by a method usually employed in this field such as extraction, for example, with a mixed solution of phenol/chloroform/isoamyl alcohol, alcohol precipitation, purification by column, filtration by filter, and the like. In addition, after such purification process, it is more preferable to extract DNA which has target base pair (bp). The extraction method includes the methods well known per se, for example, the method using liquid chromatography and the method using electrophoresis on polyacrylamide gel and the like as described in Enlarged Edition of Genetic Engineering Lab Manual, 1990, 27-28. In the case where the DNA corresponding to RNA with short strand, such as a microRNA, is intended to be refined, it is preferable to employ the electrophoretic method on polyacrylamide gel and the like, because of difficulty in separation by the liquid chromatographic method. In addition, the double-stranded DNA obtained by the PCR as described above may be subjected to further PCR reaction to obtain further larger amount.

A preferable example in this case is carried out, for example, as follows. That is, first, the solution (e.g. 10 to 20 µL) containing the single-stranded DNA involved in the present invention obtained through Steps 1 to 2 using 1 ng to 10 µg (as amount of nucleic acid) of template RNA, is passed, for example, through a filter such as a silica gel membrane, and the residual substance etc. of Steps 1 to 2 are removed. Subsequently, to the solution (e.g. 10 µL) which contains a single-stranded DNA, 0.1 to I µL of the solution containing 1 to 10 units/µL of the above-described single-stranded DNA ligase, and 0.5 to 1 µL of the solution containing 50 to 100 µmol/L of the 3'-terminal-dehydroxylated 5'-adapter involved in the present invention are added, and the solution is incubated at 50 to 60°C for 30 to 60 minutes, to obtain a 5'-adapter-bound single-stranded DNA. To the solution (e.g. 10 µL) which contains the obtained 5'-adapter-bound single-stranded DNA involved in the present invention, 0.1 to 0.5 µL of 50 to 100 µmol/L primer 1, 0.1 to 0.5 µL of 50 to 100 µmol/L primer 2, as well as 1 to 3 µL of a mixed solution containing 4 types of deoxyribonucleotide triphosphates (dNTPs) each with a concentration of 1 to 5 µmol/L, are added, and the PCR reaction is carried out, for example, by cycles of (1) 93 to 98°C for 1 to 10 minutes → (2) 93 to 98°C for 10 to 30 seconds → 50 to 60°C for 10 to 30 seconds → 68 to 72°C for 10 to 30 minutes (10 to 20 cycles), (3) 68 to 72°C for 1 to 5 minutes, and thereby an amplified double-stranded DNA can be obtained. After that, the obtained double-stranded DNA is purified by extraction, for example, with phenol/chloroform/isoamyl alcohol mixture, and the like. Further, the aforementioned double-stranded DNA is subjected, for example, to electrophoresis using non-denatured polyacrylamide gel to extract the DNA with a desired chain length. Again the extracted DNA is purified by extraction, for example, with phenol/chloroform/isoamyl alcohol mixture and the like; and thereby purified double-stranded DNA can be obtained. Further, when the double-stranded DNA is desired to be amplified, the obtained double-stranded DNA involved in the present invention may be subjected to the PCR according to the same method as the above-described PCR reaction, to obtain the amplified double-stranded DNA. By the same way as the above-described procedures after the PCR reaction, the obtained double-stranded DNA is purified by extraction, for example, with phenol/chloroform/isoamyl alcohol mixture, and the like; the DNA with a desired chain length is extracted by performing electrophoresis. Again the extracted DNA is purified by extraction, for example, with phenol/chloroform/isoamyl alcohol mixture and the like; and thereby further amplified double-stranded DNA can be obtained.

### • Method for synthesis of double-stranded DNA including 3'-adapter addition step

The method for synthesis of double-stranded DNA of the present invention further includes, prior to the above-described Step 1, a method including a step where an RNA fragment of known sequence is added to the 3'-terminal of the template RNA. Specifically, the method is performed by the following steps:
1) a step in which the 3'-adapter is added to the template RNA;
2) Step 1 in which the 3'-adapter-bound RNA is subjected to reverse transcription reaction;
3) Step 2 in which the solution processed in Step 1 is subjected to alkaline treatment;
4) Step 3 in which the obtained single-stranded DNA is converted to a double-stranded DNA.

The step in which the 3'-adapter is added to the above-described template RNA (3'-adapter addition step), Step 1 and Step 2 are the same steps as those in the paragraph of the method for synthesis of the single-stranded DNA of the present invention including the above-described 3'-adapter addition step, and preferred procedures are also the same. That is, by subjecting the single-stranded DNA involved in the present invention obtained by passing through the above-described 3'-adapter addition step, Step 1 and Step 2, to Step 3, the double-stranded DNA containing nucleotide sequence corresponding to the template RNA can be synthesized

In the above-described Step 3, the method of converting single-stranded DNA involved in the present invention to a double-stranded DNA includes the same methods as described in the paragraph of the method for synthesis of double-stranded DNA, and a preference is also given to the method by PCR. The method of forming a double strand by the PCR from a single-stranded DNA obtained by the method for synthesis of single-stranded DNA including 3'-adapter addition steps is carried out, for example, as follows.

That is, first, for example, using the single-stranded DNA ligase involved in the present invention, the 5'-adapter-bound single-stranded DNA involved in the present invention is obtained. After that, the primer 1 which is designed from the aforementioned 5'-adapter and the primer 2 which is designed from the complementary strand to the 3'-adapter are prepared, and then, using these primer 1 and primer 2, the PCR reaction is carried out for the single-stranded DNA involved in the present invention added with 5'-adapter involved in the present invention.

The single-stranded DNA ligase involved in the present invention to be employed herein includes the same one as described above in the paragraph of the method for synthesis of double-stranded DNA.

The 5'-adapter involved in the present invention may be any kinds of sequence so long as it is not complementary to the 3'-adapter, is processed so that hydroxyl group of 3'-side does not react with a phosphate group, and is known DNA. The one which consists of a sequence not existing in the single-stranded DNA involved in the present invention nor in the complementary strand thereto is preferable. Number of nucleotides thereof is usually 12 to 30, preferably 15 to 25, and more preferably 18 to 22. Treatment by which the above-described hydroxyl group of 3'-side does not react with a phosphate group includes the same one as described above in the paragraph of the method for synthesis of double-stranded DNA.

The above-described primer 1 is the one which comprises the entire or a part of complementary strand to the 5'-adapter involved in the present invention, and number of nucleotides thereof is usually 12 to 30, preferably 15 to 25, and more preferably 18 to 22. The above-described primer 2 is the one which comprises the entire or a part of complementary strand to the complementary strand of the 3'-adapter, and number of nucleotides thereof is usually 12 to 30, preferably 15 to 25, and more preferably 18 to 22. These primers 1 and 2 which have sequences not existing in the single-stranded DNA involved in the present invention nor in the complementary strand thereof are preferable.

In the process of converting a single-stranded DNA involved in the present invention obtained by the method for synthesis of single-stranded DNA including 3'-adapter addition step to a double-stranded DNA by the PCR, method of adding 5'-adapter involved in the present invention to the 5'-terminal of single-stranded DNA involved in the present invention, treatment before the addition of 5'-adapter involved in the present invention, inactivation of DNA ligase and the like after the addition, aspects of the PCR reaction and preferable aspects thereof include the same ones as described in the paragraph of the above-described method for synthesis of double-stranded DNA.

A preferable example of the method of converting a single-stranded DNA involved in the present invention obtained by the method for synthesis of single-stranded DNA including 3'-adapter addition step to a double strand by the PCR is carried out, for example, as follows.

That is, first, the solution (e.g. 10 µL) which comprises the single-stranded DNA involved in the present invention obtained through the Steps 1 to 2 using 1 ng to 1 µg (as amount of nucleic acid) of the template RNA is allowed to react with 0.5 to 3 µL of a solution containing 0.1 to 1 units/µL of dephosphorylating enzyme such as, for example, SAP, for example, at 30 to 40°C for 15 to 30 minutes, and thereby, 5'-terminal of the single-stranded DNA involved in the present invention is dephosphorylated. After that, the dephosphorylating enzyme such as SAP is inactivated by incubation at 65 to 80°C for15 to 30 minutes. Subsequently, to the solution (e.g. 10 µL) which comprises the 5'-terminal-dephosphorylated single-stranded DNA, 0.1 to 1 µL of a solution containing 1 to 10 units/µL of the above-described single-stranded DNA ligase, and 0.5 to 1 µL of a solution containing 50 to 100 µmol/L of the 3'-terminal-dehydroxylated 5'-adapter involved in the present invention are added, and the solution is incubated at 50 to 60°C for 30 to 60 minutes, to obtain the 5'-adapter-bound single-stranded DNA. To the solution (e.g. 10 µL) which comprises the obtained 5'-adapter-bound single-stranded DNA involved in the present invention, 0.1 to 0.5 µL of 50 to 100 µmol/L primer 1, 0.1 to 0.5 µL of 50 to 100 µmol/L primer 2, as well as 1 to 3 µL of a mixed solution containing 4 types of deoxyribonucleotide triphosphates (dNTPs) each with a concentration of 1 to 5 µmol/L are added, and the PCR reaction is carried out, for example, by cycles of (1) 93 to 98°C for 1 to 10 minutes → (2) 93 to 98°C for 10 to 30 seconds → 50 to 60°C for 10 to 30 seconds → 68 to 72°C for 10 to 30 minutes (10 to 20 cycles), (3) 68 to 72°C for 1 to 5 minutes, and thereby an amplified double-stranded DNA can be obtained. After that, the obtained double-stranded DNA is purified by extraction, for example, with phenol/chloroform/isoamyl alcohol mixture, and the like. Further, the aforementioned double-stranded DNA is subjected, for example, to electrophoresis using non-denatured polyacrylamide gel to extract the DNA with a desired chain length. Again the extracted DNA is purified by extraction, for example, with phenol/chloroform/isoamyl alcohol mixture and the like, and thereby a purified double-stranded DNA can be obtained. Further, when the double-stranded DNA involved in is desired to be amplified, the obtained double-stranded DNA is subjected to the PCR by the same method as the above-described PCR reaction, to obtain an amplified double-stranded DNA. By the same way as the above-described procedures after the PCR reaction, the obtained double-stranded DNA is purified by extraction, for example, with phenol/chloroform/isoamyl alcohol mixture, and the like to extract the DNA with a desired chain length by performing electrophoresis. Again the extracted DNA is purified by extraction, for example, with phenol/chloroform/isoamyl alcohol mixture and the like, and thereby further amplified double-stranded DNA. can be obtained.

As described above, the double-stranded DNA obtained by the method for synthesis of double-stranded DNA of the present invention can be analyzed for nucleotide sequence thereof by a method usually employed in this field as described in Proceedings of the National Academy of Sciences, 1995, Vol 92, 4347-4351, and so on. Specifically, for example, the double-stranded DNA obtained using the kit for cloning is transformed into competent cell and the like, then cultured, and amplified by colony PCR. After that, plasmid is extracted, and using the obtained plasmid as a template, decoding of nucleotide sequence is carried out using a kit etc. Identification of RNA can also be performed by conducting homology search employing the decoded sequence.

In addition, main outlines of the method for synthesis of the present invention are shown in Figs. 1 and 2.

### • A kit for synthesis of single-stranded DNA

A kit for synthesis of single-stranded DNA of the present invention is used for performing the method for synthesis of single-stranded DNA of the present invention as described above. Such kit comprises I) an enzyme which is capable of adding an RNA fragment to a single-stranded RNA and III) a reverse transcriptase.

The enzyme of I) which is capable of adding an RNA fragment to a single-stranded RNA includes the same one as described in the description of single-stranded RNA ligase involved in the present invention, and specifically, RNA ligase such as T4 RNA ligase, DNA ligase such as thermostable single-stranded DNA ligase as described in the literature such as Nucleic Acids Research, 2005, Vol. 33, No.1 135-142 are included. Among them, the thermostable single-stranded DNA ligase as described in the above-described literature is preferable. The aforementioned enzyme is preferable to be dissolved in a buffer solution such as HEPES, MOPS, and is provided as a solution of usually 1 to 10 units/µL, preferably 5 to 10 units/µL.
The reverse transcriptase of III) includes the same reverse transcriptase as used in the Step 1, and a preferable one is also the same. The aforementioned enzyme is preferable to be dissolved in a buffer solution such as Tris-HCl, etc., and is usually provided as a solution of 50 to 200 units/µL, preferably 100 to 200 units/µL.

The kit for synthesis of single-stranded DNA of the present invention can be prepared by adding reagents other than the above-described I) and III). Examples of such reagents are, for example, at least one type selected from the following II) and IV) to X):
II) an RNA fragment of known sequence;
IV) a primer for reverse transcription reaction;
V) a mixture of 4 kinds of deoxyribonucleotide triphosphates (dNTPs);
VI) an enzyme which is capable of dephosphorylating 5'-terminal phosphate group of RNA;
VII) a DNA fragment of known sequence;
VIII) an enzyme which is capable of adding a DNA fragment to a single-stranded DNA;
IX) an enzyme which is capable of dephosphorylating 5'-terminal phosphate group of DNA; and
X) an alkali or an aqueous solution thereof.

As for the aforementioned kit, the one which comprises any one or more sorts of an RNA fragment of II), a primer of IV), and dNTPs of V) in addition to the above-described I) and III) is preferable; and the one which further comprises the enzyme of VI) is more preferable; and the one which further comprises one or more sorts of a DNA fragment of VII), an enzyme of VIII), and an enzyme of IX) is still more preferable; and further, the one which further comprises an alkali or an aqueous solution thereof of X) is particularly preferable.

The reagent included in the kit of the present invention for synthesis of single-stranded DNA may include buffering agent such as HEPES buffer, Tris-HCl buffer and MOPS buffer, and stabilizer such as DTT and BSA as usually employed in this field, or otherwise, these buffering agents and stabilizing reagents may be included as a separate reagent and those may be mixed at the time of use. Concentration, pH, etc. of these reagents, may also be set to those commonly employed. In addition, these reagents may be freeze-dried materials.

The RNA fragment of known sequence of the above-described II) includes the RNA fragment of known sequence in the method for synthesis of the present invention, i.e., the same one as the 3'-adapter involved in the present invention, and the same can be said for the treatment method and preferable one. Such a reagent of RNA fragment of known sequence is preferably dissolved in sterilized distilled water or a buffer solution such as Tris-HCl buffer, and is usually provided as a solution of 10 to 100 µmol/L, and preferably 50 to 100 µmol/L.

The primer for reverse transcription reaction of IV) includes the same primer for reverse transcription reaction described in the method for synthesis of single-stranded DNA and the method for synthesis of single-stranded DNA including 3'-adapter addition step, and the same can be said for the preferable one. However, when the reagents kit comprises the RNA fragment of known sequence of II), the primer which comprises a sequence complementary to the aforementioned RNA fragment is preferable. The primer is preferably dissolved in sterilized distilled water or a buffer solution such as Tris-HCl buffer, and is usually provided as a solution of 10 to 100 µmol/L, and preferably 10 to 50 µmol/L.

The mixture of deoxyribonucleotides triphosphates (dNTPs) of V) is a mixture of 4 kinds of deoxyribonucleotides triphosphates comprising adenine, thymine, cytosine and guanine (dATP, dTTP, dCTP, dGTP). The mixture is preferably dissolved in sterilized distilled water or a buffer solution such as Tris-HCl buffer, and is usually provided as a solution of 1 to 10 mmol/L, and preferably 2 to 5 mmol/L.

The enzyme which is capable of dephosphorylating the 5'-terminal phosphate group of RNA of VI) includes the same enzyme as used in the treatment so that the phosphate group of 5'-terminal side of template RNA does not react with hydroxyl group in the 3'-adapter addition step, and the same can be said for the preferable one. Preferably, the aforementioned enzyme is usually dissolved in a buffer solution, such as HEPES, MOPS, and is usually provided as a solution of 0.1 to 1 units/µL, and preferably 0.5 to 1 units/ L.

The DNA fragment of known sequence of VII) includes the same one as the 5'-adapter involved in the present invention, and treatment method and preferable one are also the same. Such a reagent of DNA fragment of known sequence is preferably dissolved in sterilized distilled water or a buffer solution such as Tris-HCl buffer, and is provided usually as a solution of 10 to 100 [µmol/L, and preferably 50 to 100 µmol/L.

The enzyme which is capable of adding DNA fragment to single-stranded DNA of VIII) includes the same one as the single-stranded DNA ligase involved in the present invention, and a preferable one is also the same. It should be noted that, the aforementioned enzyme includes also the enzyme which is capable of binding a single-stranded RNA and a single-stranded RNA as well as a single-stranded DNA and a single-stranded DNA. When the enzyme having such activity is employed, the same enzyme as the one which is capable of adding RNA fragment to single-stranded RNA of I) can be used. This case is preferable because one kind of the reagents can be reduced. The enzyme which possesses such activity includes, for example, thermostable single-stranded DNA ligase and the like as described in the literature such as Nucleic Acids Research, 2005, Vol. 33, No.1 135-142. The aforementioned enzyme is preferably dissolved in a buffer solution, such as HEPES, MOPS, and is provided usually as a solution of 1 to 10 units/µL, and preferably 5 to 10 units/µL.

The enzyme which is capable of dephosphorylating the 5'-terminal phosphate group of DNA of IX) includes the same one as the enzyme which is capable of dephosphorylating the 5'-terminal phosphate group of RNA, and a preferable one is also the same. For the enzyme of VI) and the enzyme of IX), either the same enzyme or a different enzyme may be used, but the same enzyme is preferable, because one kind of reagent can be reduced by employing the same enzyme.

The alkali or an aqueous solution thereof of X) includes the same alkali or an aqueous solution thereof described in the paragraph of alkaline treatment of Step 2, and a preferable one is also the same. The aforementioned alkaline metal salt or concentration of an aqueous solution thereof may be the one which can give pH of usually 10 to 14, preferably 12 to 14 by addition in the reaction, and is provided directly or as an aqueous solution thereof of pH 14 or higher.

The kit for synthesis of single-stranded DNA of the present invention may include a known RNA as reference standard, and the aforementioned RNA is not particularly limited so long as it is known RNA. Number of nucleotides thereof is usually 15 to 30, preferably 20 to 25. The aforementioned standard is preferably dissolved in sterilized distilled water or a buffer solution such as Tris-HCl buffer, and is provided usually as a solution of 10 to 100 ng/µL, and preferably 10 to 50 ng/µL.

The kit for synthesis of single-stranded DNA of the present invention may take a form which further comprises RNase inhibitor, manganese chloride, EDTA, carrier for ethanol precipitation, ammonium acetate, etc. which are used in the method for synthesis of single-stranded DNA of the present invention, and all of those usually employed in this field can be used, and their concentrations may also be set to the concentrations usually used.

A preferable example of the kit for synthesis of single-stranded DNA of the present invention includes, for example, the one which comprises II) an RNA fragment of known sequence, III) a reverse transcriptase, IV) a primer for reverse transcription reaction, V) dNTPs, VI) an enzyme which is capable of dephosphorylating the 5'-terminal phosphate group of RNA, VII) a DNA fragment of known sequence, IX) an enzyme which is capable of dephosphorylating the 5'-terminal phosphate group of DNA and X) an alkali or an aqueous solution thereof.

### • A kit for synthesis of double-stranded DNA

The kit for synthesis of double-stranded DNA of the present invention is used for performing the method for synthesis of double-stranded DNA of the present invention as described above. Such kit comprises I) an enzyme which is capable of adding an RNA fragment to a single-stranded RNA, II) an RNA fragment of known sequence, III) a reverse transcriptase, IV) a primer for reverse transcription reaction, V) dNTPs, VI) an enzyme which is capable of dephosphorylating the 5'-terminal phosphate group of RNA, VII) a DNA fragment of known sequence, VIII) an enzyme which is capable of adding a DNA fragment to a single-stranded DNA, IX) an enzyme which is capable of dephosphorylating the 5'-terminal phosphate group of DNA, and XI) a nucleotide sequence corresponding to the template RNA comprising PCR primer, and the kit which further comprises X) an alkali or an aqueous solution thereof is preferable.

The kit for synthesis of single-stranded DNA of the present invention may include a buffering agent such as HEPES buffer, Tris-HCl buffer, MOPS buffer and imidazole buffer and a stabilizer such as DTT and BSA as usually employed in this field. These buffering agent and stabilizing agent may be added as separate reagents, which are mixed at the time of use. Concentration, pH, etc. of these reagents, may also be set to the one commonly employed. In addition, these reagents may be freeze-dried materials.

The enzyme of I), the RNA fragment of known sequence of II), the reverse transcriptase of III), the primer for reverse transcription reaction of IV), the dNTPs of V), the enzyme which is capable of dephosphorylating the 5'-terminal phosphate group of RNA of VI), the DNA fragment of known sequence of VII), the enzyme which is capable of adding DNA fragment to single-stranded DNA of VIII), the enzyme which is capable of dephosphorylating the 5'-terminal phosphate group of DNA of IX) and the alkali or an aqueous solution thereof of X) are the same as those described in the paragraph of the above-described kit for synthesis of single-stranded DNA.

It should be noted that, the enzyme of I) which is capable of adding an RNA fragment to a single-stranded RNA and the enzyme of VIII) which is capable of adding a DNA fragment to a single-stranded DNA include the enzyme which is capable of binding a single-stranded RNA and a single-stranded RNA as well as a single-stranded DNA and a single-stranded DNA, and therefore, in the case where both of the enzymes are employed together, the enzymes of I) and VIII) may be contained in one reagent. In addition, with respect to the enzyme of VI) which is capable of dephosphorylating 5'-terminal phosphate group of RNA and the enzyme of IX) which is capable of dephosphorylating 5'-terminal phosphate group of DNA, when those enzymes are employed, the enzymes may be unified as one reagent using either reagent VI) or IX).

The PCR primer of XI) consists of the primer 1 and the primer 2 which are described in the method of converting single-stranded DNA to double-stranded DNA by the PCR in Step 3, and the primer 1 and the primer 2 include the same ones as those described in the method of converting single-stranded DNA to double-stranded DNA by the PCR. The above-described primer 1 is preferably dissolved usually in sterilized distilled water or a buffer solution such as Tris-HCl buffer, and usually provided as a solution of 10 to 100 µmol/L, and preferably 50 to 100 µmol/L. The above-described primer 2 is preferably dissolved usually in sterilized distilled water or a buffer solution such as Tris-HCl buffer, and usually provided as a solution of 10 to 100 µmol/L, and preferably 50 to 100 µmol/L. These primers 1 and 2 may be provided as a mixture, however, preferably provided as a separate reagent.

The kit for synthesis of double-stranded DNA of the present invention may include known RNA as a reference standard, and the aforementioned RNA is not particularly limited, so long as it is known RNA. The aforementioned standard is preferably dissolved in sterilized distilled water or a buffer solution such as Tris-HCl buffer, and is provided usually as a solution of 10 to 100 ng/µL, and preferably 10 to 50 ng/µL.

The kit for synthesis of double-stranded DNA of the present invention may take a form which further comprises RNase inhibitor, manganese chloride, EDTA, carrier for ethanol precipitation, ammonium acetate, etc., and all of those usually employed in this field can be used, and their concentrations may also be set to those usually used.

A preferable example of the kit for synthesis of double-stranded DNA of the present invention includes, for example, the one which comprises II) an RNA fragment of known sequence, III) a reverse transcriptase, IV) a primer for reverse transcription reaction, V) dNTPs, VI) an enzyme which is capable of dephosphorylating the 5'-terminal phosphate group of RNA, VII) a DNA fragment of known sequence, IX) an enzyme which is capable of dephosphorylating 5'-terminal phosphate group of DNA and XI) a PCR primer.

Hereinafter, the present invention will be explained in detail by referring to Examples and Reference Examples, however, the present invention is not limited thereto in any way.

### [Example]

### Example 1: Cloning of small RNA

### 1. Extraction of small RNA fraction

HeLa cells (Dainippon Pharmaceutical Co., Ltd.), 1 x 10⁷ cells, were dissolved by adding 1 mL of ISOGEN (Nippon Gene Co., Ltd.), then 0.3 mL of chloroform was added thereto, and centrifugal separation was carried out (14,000 rpm (18,800 x g), 10 minutes). The separated aqueous phase was extracted, and the same volume of isopropanol was added to precipitate RNA, and thus HeLa total RNA was obtained.

Subsequently, using mirVana^{™} miRNA Isolation Kit (Ambion, Inc.), and according to the method described in the attached manual, the small RNA fraction comprising 200 or less nucleotides was extracted from the obtained HeLa total RNA. As a result, 15.4 µg of the small RNA comprising 200 or less nucleotides was obtained.

Further, the total amount (15.4 µg) of the obtained small RNA fraction comprising 200 or less nucleotides was introduced into 15% denatured polyacrylamide gel and electrophorated (constant voltage, 30 mA). After carrying out the electrophoresis, an area of the gel corresponding to 20 to 24 nucleotides was cut out, and using small RNA Gel Extraction Kit (Takara Biotech Co., Ltd.), RNA having 20 to 24 nucleotides (microRNA) was collected according to the method described in the attached instruction manual. An image of the above-described gel after electrophoresis is shown in Fig. 3.

### 2. Dephosphorylation reaction of microRNA

The microRNA solution, 15 µL (total amount), obtained as the above, was heated at 70°C for 3 minutes, and then allowed to stand on ice for 2 minutes. After that, 1 µL of SAP solution (1 unit/µL, shrimp derived alkaline phosphatase: United States Biological Inc.) and 4 µL of SAP reaction buffer (500 mmol/L HEPES buffer solution containing 50 mmol/L magnesium chloride, 100 mmol/L potassium chloride (pH 7.3)) were added thereto, and mixed gently under ice-cooling state; and the mixture was further incubated at 37°C for 15 minutes, the SAP was inactivated by heating at 65°C for 15 minutes; and then allowed to stand on ice for 2 minutes.

### 3. Adapter binding to 3'-terminal of microRNA

To the obtained solution, 15 µL of sterilized water, 1 µL of 50 µmol/L adapter (SEQ ID NO: 1: 5'-P-AAGAAGCUGGCGUCAAUGU-2'3'ddC-3'), 1 µL of 10 mmol/L magnesium chloride, 1 µL of reaction solution for ThermoPhage Single-Stranded DNA Ligase [2 mmol/L adenosine 5'-triphosphate tetrasodium (ATP), 40 mmol/L dithiothreitol, 1 µg/µL bovine serum albumin (BSA)], 1 µL of Ribonuclease Inhibitor (Super) solution (20 units/µL: Wako Pure Chemical Industries Ltd.) and 1 µL of ThermoPhage Single-Stranded DNA Ligase solution (10 units/µL: PROKARIA Ltd.) were added, and mixed gently under ice-cooling condition. Subsequently, after the mixture was incubated at 60°C for 30 minutes and the thermostable single-stranded DNA ligase was inactivated by heating at 90°C for 5 minutes, the mixture was allowed to stand on ice for 2 minutes. Then, to the reaction solution, 120 µL of sterilized water was added and mixed, and further the same amount (160 µL) of mixed solution of phenol/chloroform/isoamyl alcohol (25:24:1) (Nippon Gene Co., Ltd.) was added and mixed using vortex mixer. Then the mixture was centrifuged at 4°C, 14,000 rpm (18,800 x g) for 5 minutes, and water layer (upper layer) was harvested. Subsequently, to the water layer, 1 µL of Ethachinmate (Nippon Gene Co., Ltd.), 1/4 volume of the obtained aqueous layer of 10 mmol/L ammonium acetate, twofold of total volume of 99.5% ethanol were added and mixed using vortex mixer; and the mixture was allowed to stand at room temperature for 30 minutes or more. Again, the reaction mixture was subjected to the centrifugal separation at 4°C, 14,000 rpm (18,800 x g) for 15 minutes; the supernatant solution was removed by aspiration carefully so as not to lose precipitate; the precipitate was washed twice with 70% ethanol and dried; the dried precipitate was dissolved in 11 µL of sterilized water, and thus the obtained microRNA whose 3'-terminal had been bounded with an adapter was purified.

### 4. Reverse Transcription (RT) Reaction

To the total amount of solution (11 µL) obtained in 3, 1 µL of 50 µmol/L primer (SEQ ID NO: 2: 5'-GACATTGACGCCAGCTTCTT-3') was added and the solution was heated at 70°C for 3 minutes, and the reaction mixture was allowed to stand on ice for 2 minutes. Two µL of the buffer solution for reverse transcription reaction [500 mmol/L Tris-HCl buffer (pH 8.3) containing 750 mmol/L potassium chloride, 30 mmol/L magnesium chloride and 50 mmol/L dithiothreitol], 4 µL of dNTPs (mixed solution comprising each 2.5 mmol/L dATP, dGTP, dCTP and dTTP: Nippon Gene Co., Ltd.), 1 µL of Ribonuclease Inhibitor (Super) solution (20 uits/µL: Wako Pure Chemical Industries Ltd.) and reverse transcriptase (200 uits/µL ReverScriptIV: Wako Pure Chemical Industries Ltd.) were added thereto, and the solution was mixed gently under ice-cooling condition. After incubation at 42°C for 30 minutes, 2 µL of 0.5 mol/L EDTA was added thereto and mixed well to terminate the reaction.

### 5. Alkaline treatment

To the solution after termination of the reaction, 8 µL of 0.2 mol/L sodium hydroxide solution was added, and the solution was heated at 65°C for 30 minutes, then allowed to stand on ice for 2 minutes, and thereby, RNA used as a template of reverse transcription reaction etc. was hydrolyzed. Further, 20 µL of 1 mol/L Tris-HCl (pH 7.5) was added thereto, and mixed using vortex mixer, then 110 µL of sterilized water was added thereto, and mixed using vortex mixer. Further, the same amount (160 µL) of mixed solution of phenol/chloroform/isoamyl alcohol (25:24:1) was added and mixed using vortex mixer. Subsequently, the mixture was centrifuged at 4°C, 14,000 rpm (18,800 x g) for 5 minutes, and water layer (upper layer) was harvested. Further, to this water layer, 1 µL of Ethachinmate (Nippon Gene Co., Ltd.), 1/4 volume of the harvested aqueous layer of 10 mmol/L ammonium acetate, twofold of total volume of 99.5% ethanol were added and mixed using vortex mixer; and the mixture was allowed to stand at room temperature for 30 minutes or more. The reaction mixture was subjected to the centrifugal separation at 4°C, 14,000 rpm (18,800 x g) for 15 minutes; the supernatant solution was removed; the precipitate was washed twice with 70% ethanol and dried; the dried precipitate was dissolved in 15 µL of sterilized water.

### 6. Dephosphorylation reaction of the solution after RT reaction

The total amount of the solution obtained in 5 was subjected to heat treatment at 90°C for 5 minutes, then allowed to stand on ice for 2 minutes. Subsequently, 4 µL of SAP reaction buffer [500 mmol/L HEPES buffer solution containing 50 mmol/L magnesium chloride, 100 mmol/L potassium chloride (pH 7.3)] and 1 µL of 1 unit/µL SAP solution (shrimp derived alkaline phosphatase: United States Biological Inc.) were added thereto and the solution was mixed gently under ice-cooling state; and after incubation at 37°C for 15 minutes, the SAP was inactivated by heating at 80°C for 15 minutes; and then the mixture was allowed to stand on ice for 2 minutes.

### 7. Binding of an adapter to the 3'-terminal of reverse transcription reaction product

To the solution obtained in 6, 16 µL of sterilized water, 1 µL of 50 µmol/L adapter (SEQ ID NO: 3: 5'-P-AAGGCTCAGTCTCGGGATA-2'3'ddC-3'), 1 µL of 10 mmol/L magnesium chloride, 1 µL of reaction solution for ThermoPhage Single-Stranded DNA Ligase [2 mmol/L adenosine 5'-triphosphate tetrasodium (ATP), 40 mmol/L dithiothreitol, 1 µg/µL bovine serum albumin (BSA)], and I µL of ThermoPhage Single-Stranded DNA Ligase solution (10 units/µL: PROKARIA Ltd.) were added, and the mixture was mixed gently under ice-cooling condition. Subsequently, after the mixture was incubated at 60°C for 30 minutes, and the thermostable single-stranded DNA ligase was inactivated by heating at 90°C for 5 minutes, the mixture was allowed to stand on ice for 2 minutes. To the total amount of reaction solution, 120 µL of sterilized water was added and mixed using vortex mixer, and further the same amount (160 µL) of mixed solution of phenol/chloroform/isoamyl alcohol (25:24:1) (Nippon Gene Co., Ltd.) was added and mixed using vortex mixer. Subsequently, the mixture was centrifuged at 4°C; 14,000 rpm (18,800 x g) for 5 minutes, and water layer (upper layer) was harvested. After that, to the water layer, 1 µL of Ethachinmate (Nippon Gene Co., Ltd.), 1/4 volume of the recovered aqueous layer of 10 mmol/L ammonium acetate, twofold of the total volume of 99.5% ethanol were added and mixed using vortex mixer; and the mixture was allowed to stand at room temperature for 30 minutes or more. Subsequently, the reaction mixture was subjected to the centrifugal separation at 4°C, 14,000 rpm (18,800 x g) for 15 minutes; the supernatant solution was removed and the precipitate was washed twice with 70% ethanol. After that, the precipitate was dried and then dissolved in 33.5 µL of sterilized water.

### 8. PCR (the 1st time)

To the entire amount of solution obtained in 7, 5 µL of 10 x Reaction buffer (Eurogentec S.A.), 5 µL of dNTPs (mixed solution comprising each 2.5 mmol/L dATP, dGTP, dCTP and dTTP: Nippon Gene Co., Ltd.), 1 µL of 50 µmol/L primer (SEQ ID NO: 4: 5'-GTATCCCGAGACTGAGCC-3'), 1 µL of 50 µmol/L primer (SEQ ID NO: 5: 5'-GACATTGACGCCAGCTTC-3'), 4 µL of 25 mmol/L magnesium chloride, 0.5 µL of HOTGoldstar^{™} DNA Polymerase solution (5 units/µL: Eurogentec S.A.) were added and mixed gently under ice-cooling condition, and the PCR reaction was carried out under the following conditions:
95°C for 10 minutes → 95°C for 20 seconds · 56°C for 10 seconds · 72°C for 10 seconds (15 cycle) → 72°C for 1 minute.

After standing the obtained solution on ice for 2 minutes, 110 µL of sterilized water was added, and further the same amount (160 µL) of mixed solution of phenol/chloroform/isoamyl alcohol (25:24:1) (Nippon Gene Co., Ltd.) was added and mixed using vortex mixer. Subsequently, the mixture was centrifuged at 4°C; 14,000 rpm (18,800 x g) for 5 minutes, and water layer (upper layer) was harvested. To the obtained water layer, 1 µL of Ethachinmate (Nippon Gene Co., Ltd.), 1/4 volume of the recovered aqueous layer of 10 mmol/L ammonium acetate, twofold of the total volume of 99.5% ethanol were added and mixed using vortex mixer; and the mixture was allowed to stand at room temperature for 30 minutes or more. Subsequently, the mixture was centrifuged at 4°C; 14,000 rpm (18,800 x g) for 5 minutes; the supernatant was removed carefully so as not to aspirate the precipitate; the precipitate was washed twice with 70% ethanol; dried at 45°C or lower; dissolved in 10 µL of sterilized water; then 2 µL of 6 x Loading Buffer Triple Dye (Nippon Gene Co., Ltd.) was added thereto and mixed.

### 9. Electrophoresis (the 1st time)

Electrophoresis (constant current, 30 mA) was performed for the solution obtained in 8 on a non-denatured 15% polyacrylamide gel (SuperSep: Wako Pure Chemical Industries Ltd.). In addition, as a molecular weight marker, 10bp DNA Step Ladder (Wako Pure Chemical Industries Ltd.) was employed, and as an electrophoresis buffer, the solution which was prepared by dissolving 3.1 g of Tris (hydroxymethyl) aminomethane and 14.5 g of Glycine in distilled water and filled up to 1 L was used. The electrophoresis was stopped at the time when Xylene cyanol (XC: Wako Pure Chemical Industries Ltd.) which was mixed in advance with the solution obtained in 7 has moved out from the bottom of the electrophoresis gel, and the gel was stained by soaking for 10 minutes in a staining solution which was prepared by dissolving 0.5 µg/mL of ethidium bromide in the electrophoresis buffer. Then, after washing the gel with sterilized water for 2 to 3 minutes, the gel was observed using FAS-III (ultraviolet light irradiator: Toyobo Co., Ltd.), and the portion (60 to 64 bp) of the target chain length was cut out. Subsequently, a filter tube of BioMasher (Wako Pure Chemical Industries Ltd.) was set to a 1.5 mL microtube, and the gel cut out was inserted into the filter tube.

A solution (1 x M Buffer, 100 µL) which was prepared by diluting 10 x M Buffer (100 mmol/L Tris-HCl buffer solution (pH 7.5) containing 500 mmol/L sodium chloride, 100 mmol/L magnesium chloride, 10 mmol/L DTT: Nippon Gene Co., Ltd.) with sterilized water to 1/10 was added to the filter tube. Then, the crushing stick was inserted from the top, and rotated toward right and left while the stick was pushed against the filter tube to mash up the gel. Further, after carrying out centrifugal separation at 4°C, 14,000 rpm (18,800 x g) for 5 minutes, the crushing stick was pulled out from the filter tube and 100 µL of 1 x M Buffer was added to the filter tube. In addition, the gel adhering to the crushing stick was exfoliated by forceps etc., and was incorporated with the filter tube. After allowing to stand at room temperature for 1 hour, the crushing stick was inserted from the top, and rotated toward right and left while the stick was pushed against the filter tube to mash up the gel, and the filter tube was subjected to centrifugal separation at 4°C; 14,000 rpm (18,800 x g) for 5 minutes. The obtained supernatant solution was harvested, and the same amount of mixed solution of phenol/chloroform/isoamyl alcohol (25:24:1) was added thereto and mixed using vortex mixer. Further, the mixture was subjected to centrifugal separation at 4°C; 14,000 rpm (18,800 x g) for 5 minutes, and water layer (upper layer) was harvested. After that, to the water layer, 1 µL of Ethachinmate (Nippon Gene Co., Ltd.), 1/4 volume of the recovered aqueous layer of 10 mmol/L ammonium acetate, twofold of the total volume of 99.5% ethanol were added and mixed using vortex mixer; and the mixture was allowed to stand at room temperature for 30 minutes or more. Subsequently, the mixture was centrifuged at 4°C; 14,000 rpm (18,800 x g) for 5 minutes; the supernatant was removed carefully so as not to aspirate the precipitate; the precipitate was washed twice with 70% ethanol; dried at 45°C or lower; the precipitate was dissolved in 33.5 µL of sterilized water.

### 10. PCR (the 2nd time)

The total amount of the obtained solution was transferred to a PCR tube, then the same operations were carried out as in 8. PCR (in the 1st time).

### 11. Electrophoresis (the 2nd time)

The solution obtained in 10, each 6 µL per one lane, was introduced into the non-denatured 15% polyacrylamide gel (SuperSep), electrophoresis was carried out by the same procedures as in 9.

After drying the obtained solution under 45°C or lower, the precipitate was dissolved in 5 µL of sterilized water, and cDNA for cloning was obtained. The image of the gel after electrophoresis is shown in Fig. 4.

### 12. Transformation

To the cDNA (2 µL) for cloning obtained in 11, 1 µL of 20 ng/µL pGEM Teasy Vector (Promega KK) and 3 µL of DNA Ligation Kit Mighty Mix solution (TAKARA Biotech Co., Ltd.) were added and the solution was incubated at 16°C for 30 minutes, and thereby the cDNA for cloning was inserted into the vector. Then, using ECOS^{™} Competent *E. coli* DH5 α (Nippon Gene Co., Ltd.), the total amount of the vector was transformed into the competent cell by the heat shock method at 42°C for 45 seconds. Subsequently, the cells were cultured in Luria-Bertani's broth (LB) agar medium containing 100 µg/mL ampicillin sodium at 37°C for 16 hours.

### 13. Colony PCR

To single colony on the medium, 1 µL of 10 x Universal Buffer (Nippon Gene Co., Ltd.), 1 µL of 2.5 mM dNTPs (mixed solution comprising each 2.5 mmol/L dATP, dGTP, dCTP and dTTP: Nippon Gene Co., Ltd.), 0.5 µL of 50 µmol/L primer (SEQ ID NO: 6: 5'-GTATCCCGAGACTGAGCC-3'), 0.5 µL of 50 µmol/L primer (SEQ ID NO: 7: 5'-GACATTGACGCCAGCTTC-3'), 6.9 µL of sterilized water, and 0.1 µL of Gene Taq NT (5 units/µL: Nippon Gene Co., Ltd.) were added, and the mixture was mixed gently under ice-cooling condition, and then the PCR reaction was carried out under the following condition:
95°C for 2 minutes → 95°C for 20 seconds · 56°C for 10 seconds · 72°C for 10 seconds (30 cycles) → 72°C for 1 minute.

Subsequently, to the obtained PCR reaction mixture, 1 µL of 6 x Loading Buffer Triple Dye (Nippon Gene Co., Ltd.) was added and mixed.

### 14. Electrophoresis

The solution (5 µL) obtained in 13 was loaded on a non-denatured 15% polyacrylamide gel (SuperSep: Wako Pure Chemical Industries Ltd.) and electrophoresis was carried out (by 30 mA constant current per a gel). After soaking the gel into 0.5 µg/mL of ethidium bromide staining solution for 10 minutes, the presence of insertion fragment and chain length thereof were identified using FAS-III (ultraviolet light irradiator: Toyobo Co., Ltd.).

### 15. Plasmid extraction

The single clone, in which presence of the inserted fragment had been confirmed in 14, was cultured with shaking in the LB medium containing 100 µg/mL of ampicillin sodium at 37°C for 16 hours, and then using QIAprep Spin Miniprep Kit (QIAGEN GmbH), the plasmid was extracted according to the method described in the manual attached.

### 16. Nucleotide sequence analysis

Using the plasmid (200 ng) obtained in 15 as a template, and using DYEnamic ET Terminator Cycle Sequencing Kit (GE Healthcare UK Ltd.), reaction was carried out according to the method described in the manual attached. Subsequently, determination of the nucleotide sequence was performed using BaseStaion (Bio-Rad Laboratories, Inc.).

### 17. Homology search of nucleotide sequence

Homology search (BLAST) was performed for 74 obtained sequences using DDBJ (DNA Data Bank of Japan) database. Clone distribution and microRNA distribution are shown in Table 1 and Table 2, respectively.

**[Table 1]**

| | Number of clones: nucleotide sequence determined | Component ratio (%) |
|---|---|---|
| Micro RNA | 70 | 72.9 |
| Ribosomal RNA | 21 | 21.9 |
| Transfer RNA | 3 | 3.1 |
| Others | 2 | 2.1 |
| Total | 96 | 100 |

In addition, distribution of microRNA is shown below.

**[Table 2]**

| Sanger miRNA mature name | Number of clones |
|---|---|
| Has-miR-23a | 38 |
| Has-miR-92a | 16 |
| Has-miR-22 | 5 |
| Has-miR-25 | 5 |
| Has-miR-23b | 3 |
| Has-miR-19b | 1 |
| Has-miR-21 | 1 |
| Has-miR-210 | 1 |
| Total | 70 |

From the results described above, it turned out that, according to the method of the present invention, single-stranded DNA and double-stranded DNA were easily acquirable from RNA in a sample. Consequently, it turned out that the aforementioned method is able to perform by fewer and more simple steps since there are no necessity such as gel extraction, yet a safe method because of not using RI, and a superior method as compared with conventional method, for example, the method which uses Dyna Express miRNA Cloning Kit (FUNAKOSHI NEWS June 15, 2007 issue, page 4).

In addition, as a result of obtaining and analyzing a clone using the obtained double-stranded DNA, it turned out that 72.9% of the obtained clones were cDNA of the microRNA origin. According to the conventional method, for example, the result (Table1) described in Nucleic Acids Research, 2006, Vol. 34, No. 6, only 3,374 clones of the microRNA have been obtained among 20,014 clones, i.e., only about 17% has been obtained. Therefore, it became evident from the aforementioned results that, according to the methods of the present invention, cDNA derived from short chain RNA, such as microRNA, can be obtained with higher efficiency as compared with conventional method. It is considered that this is due to alkaline treatment by which the residual RNA fragments are hydrolyzed to monomers, and also due to reduced number of step which minimize the possibility of contamination of impurities.

### Example 2: Cloning of mouse mRNA

### 1. Reverse Transcription Reaction

Total RNA (10 µg) from mouse testis (C57 Black6) was dissolved in 11 µL of sterilized water, and I µL of a primer for reverse transcription reaction (25 pmol/µL, 5'-TAGCTCTGCTCTTGGGATTG-3') which is complementary to mouse p16 INK4a mRNA was added thereto. After subjecting the mixture to heat treatment at 70°C for 3 minutes, the mixture was allowed to stand on ice for 2 minutes. Subsequently, 2 µL of 10 x reaction buffer for ReverScriptIV (Wako Pure Chemical Industries Ltd.), 4 µL of 2.5 mmol/L dNTP Mixture (Nippon Gene Co., Ltd.), 1 µL of RNase Inhibitor, Super (20 U/µL, Wako Pure Chemical Industries Ltd.), and I µL of ReverScriptIV (200 U/µL, Wako Pure Chemical Industries Ltd.) were added thereto, and mixed gently on ice. The obtained mixed solutions was incubated at 42°C for 30 minutes, and then 2 µL of 0.5 mol/L EDTA was added thereto, and the solution was mixed to terminate the reaction.

### 2. Alkaline treatment

To the solution after termination of the reaction, 8 µL of 0.2 mol/L NaOH was added and mixed, and the solution was heated for alkaline hydrolysis at 65°C for 30 minutes, then allowed to stand on ice for 2 minutes. Further, after 20 µL of I mol/L Tris-HCl (pH 7.5) was added thereto and mixed, the unreacted primer for reverse transcription reaction was removed using Invisorb Spin PCRapid Kit (Invitek GmbH). Subsequently, 1 µL of Ethachinmate (Nippon Gene Co., Ltd.), 12 µL of 10 mmol/L of ammonium acetate, and 125 µL of ethanol were each added thereto, and mixed. Further, the mixture was subjected to centrifugal separation at 18,800 x g for 10 minutes, and the precipitate was washed twice with 75% ethanol and dried, then dissolved in 16 µL of sterilized water.

### 3. Binding of adapter to reverse transcription reaction product

To the solution after the alkaline treatment, 16 µL of reverse transcription reaction solution was added. The solution was subjected to heat treatment at 90°C for 3 minutes to make DNA single strand, then allowed to stand on ice for 2 minutes. Subsequently, 1 µL of adaptor (20 pmol/µL, 5'-terminal-phosphorylated and 3'-terminal-2'3'ddC blocked, 5'-p-AAGCCTCAGTCTCGTCGATACCATG-ddC-3'), 2 µL of reaction liquid for thermostable single-stranded DNA ligase (500 mmol/L HEPES, pH 7.3, containing 50 mmol/L MgCl₂, 100 mmol/L KCI, 0.5 mmol/L ATP, 10 mmol/L DTT, and 0.25 µg/µL BSA), 1 µL of Single Strand DNA Ligase solution (10 U/µL: Wako Pure Chemical Industries Ltd.) were added thereto, and mixed gently on ice. The mixture was incubated at 60°C for 30 minutes, and further heated at 90°C for 5 minutes, thereby the Single Strand DNA Ligase was inactivated. Then, the mixture was left standing on ice for 2 minutes, and 30 µL of sterilized water was added thereto, and mixed. Furthermore, after removing unreacted adapter using Invisorb Spin PCRapid Kit (Invitek GmbH), I µL of Ethachinmate (Nippon Gene Co., Ltd.), 12 µL of 10 mmol/L ammonium acetate, and 125 µL of ethanol were added, respectively, and mixed. The mixture was subjected to the centrifugal separation at 18,800 x g for 10 minutes; the precipitate was washed twice with 75% ethanol and dried; and the precipitate was dissolved in 34.5 µL of sterilized water.

### 4. PCR (the 1st time)

To an aqueous solution (34.5 µL) comprising adapter-bound reverse transcription reaction product obtained in 3, 5 µL of 10 x Reaction Buffer for HotGoldstar DNA polymerase (Eurogentec S.A.), 4 µL of 2.5 mmol/L dNTP Mixture (Nippon Gene Co., Ltd.), 1 µL of PCR primer which is complementary to the adapter (25 pmol/µL, 5'-CATGGTATCGACGAGACTGAG-3'), 1 µL of PCR primer which is complementary to mouse p16 INK4a mRNA (25 pmol/µL 5'-AACTACTCGGATCAGACATC-3'), 4 µL of 25 mmol/L MgCl₂, 0.5 µL of HotGoldstar DNA Polymerase (5 U/µL: Eurogentec S.A.) were added, respectively, and mixed gently on ice; and the PCR reaction was carried out under the following condition:
95°C for 10 minutes → 95°C for 20 seconds · 55°C for 20 seconds · 72°C for 20 seconds (30 cycles) → 72°C for 2 minutes.

Subsequently, the unreacted PCR primer was removed from the obtained solution using Invisorb Spin PCRapid Kit (Invitek GmbH). Then, 1 µL of Ethachinmate (Nippon Gene Co., Ltd.), 12 µL of 10 mmol/L ammonium acetate, and 125 µL of ethanol were added, respectively, and mixed. Further, the mixture was subjected to centrifugal separation at 18,800 x g for 10 minutes; the precipitate was washed twice with 75% ethanol and dried; and the precipitate was dissolved in 34.5 µL of sterilized water.

### 5. PCR (the 2nd time)

To an aqueous solution (34.5 µL) obtained in 4, 5 µL of 10 x Reaction Buffer for HotGoldstar DNA polymerase (Eurogentec S.A.), 4 µL of 2.5 mmol/L dNTP Mixture (Nippon Gene Co., Ltd.), 1 µL of PCR primer which is complementary to the adapter (25 pmol/µL, 5'-CATGGTATCGACGAGACTGAG-3'), 1 µL of PCR primer which is complementary to mouse p16 INK4a mRNA (25 pmol/µL 5'-AGTAATGTTCCCTCCCTATC-3'), 4 µL of 25 mmol/L MgCl₂, 0.5 µL of HotGoldstar DNA Polymerase (5 U/µL: Eurogentec S.A.) were added, respectively, and mixed gently on ice; and the PCR reaction was carried out under the following condition:
95°C for 10 minutes → 95°C for 20 seconds · 55°C for 20 seconds · 72°C for 20 seconds (30 cycles) → 72°C for 2 minutes.

Subsequently, unreacted PCR primer was removed from the obtained solution using Invisorb Spin PCRapid Kit (Invitek GmbH). Then, 1 µL of Ethachinmate (Nippon Gene Co., Ltd.), 12 µL of 10 mmol/L ammonium acetate, and 125 µL of ethanol were added, respectively, and mixed. Further, the mixture was subjected to the centrifugal separation at 18,800 x g for 10 minutes; the precipitate was washed twice with 75% ethanol and dried; and the precipitate was dissolved in 4 µL of sterilized water.

### 6. Confirmation of PCR amplification product

Confirmation of chain length of the PCR amplification product was carried out using Bioanalyzer DNA 1000 Kit (Agilent Technologies). As a result, the PCR amplification product was confirmed at the position of about 240 bp. The results are shown in Fig. 5.

### 7. Nucleotide sequence analysis

The nucleotide sequence of the above-described PCR amplification product was analyzed using BaseStaion (Bio-Rad Laboratories, Inc.).

### (Result of nucleotide sequence analysis)

### 8. Comparison with database

Nucleotide sequence of the present cDNA was anticipated from the results of analysis obtained, and compared with the EST database (ACCESSION No.BY748960) of mouse p16 INK4a which had been registered on NCBI (National Center for Biotechnology Information). In consequence, it has been confirmed that the present cDNA has about 610 bp longer 5'-terminal. That is, it has been also found that, by the method of the present invention, the cDNA which is complementary to mouse p16 INK4a can be obtained; the cDNA which is complementary to an unknown region from a known region to 5'-terminal can be sequenced; and as a result, 5'-terminal sequence of mouse p16 INK4a can be determined.

The nucleotide sequence of mouse p16 INK4a analyzed by the methods of the present invention is shown below. In addition, the underlined part was the longer part than the EST database of mouse p16 INK4a which had been registered on NCBI.

The EST (expression sequence tag) of p16 INK4a having the maximal length of 5'-terminal which had been registered in the database is shown below.

### Example 3: Cloning of human mRNA

### 1. Reverse transcription reaction

Total RNA (10 µg) from HEK 293T cell (human embryonic kidney cell) was dissolved in 11 µL of sterilized water, and 1 µL of a primer for reverse transcription reaction (25 pmol/µL, 5'-TTCTCAGAGCCTCTCTGGTT-3') which is complementary to human p16 INK4a mRNA was added thereto; and after subjecting the mixture to heat treatment at 70°C for 3 minutes, the mixture was allowed to standing on ice for 2 minutes. Subsequently, 2 µL of 10 x reaction buffer for ReverScriptIV (Wako Pure Chemical Industries Ltd.), 4 µL of 2.5 mmol/L dNTP Mixture (Nippon Gene Co., Ltd.), 1 µL of RNase Inhibitor, Super (20 U/µL, Wako Pure Chemical Industries Ltd.), and I µL of ReverScriptIV (200 U/µL, Wako Pure Chemical Industries Ltd.) were added thereto, and mixed gently on ice. The obtained mixed solutions was incubated at 42°C for 30 minutes, and then 2 µL of 0.5 mol/L EDTA was added thereto and mixed to terminate the reaction.

### 2. Alkaline treatment

To the solution after termination of the reaction, 8 µL of 0.2 mol/L NaOH solution was added and mixed, and the solution was heated at 65°C for 30 minutes, then allowed to stand on ice for 2 minutes. Further, after 20 µL of 1 mol/L Tris-HCl (pH 7.5) was added thereto and mixed, the unreacted primer for reverse transcription reaction was removed using Invisorb Spin PCRapid Kit (Invitek GmbH). Subsequently, 1 µL of Ethachinmate (Nippon Gene Co., Ltd.), 12 µL of 10 mmol/L ammonium acetate, and 125 µL of ethanol were added, respectively, thereto, and mixed. Further, the mixture was subjected to centrifugal separation at 18,800 x g for 10 minutes, and the precipitate was washed twice with 75% ethanol and dried, then dissolved in 16 µL of sterilized water.

### 3. Binding of adapter to reverse transcription reaction product

To the solution after the alkaline treatment, 16 µL of reverse transcription reaction mixture was added, and the solution was subjected to heat treatment at 90°C for 3 minutes to make DNA single stranded, then allowed to stand on ice for 2 minutes. Subsequently, 1 µL of adaptor (20 pmol/µL, 5'-terminal-phosphorylated and 3'-terminal-2'3'ddC blocked, 5'-p-AAGCCTCAGTCTCGTCGATACCATG-ddC-3'), 2 µL of reaction liquid for thermostable single-stranded DNA ligase (500 mmol/L HEPES, pH 7.3, containing 50 mmol/L MgCl₂, 100 mmol/L KCl, 0.5 mmol/L ATP, 10 mmol/L DTT, and 0.25 µg/µL BSA), 1 µL of Single Strand DNA Ligase solution (10 U/µL: Wako Pure Chemical Industries Ltd.) were added thereto, and mixed gently on ice. The mixture was incubated at 60°C for 30 minutes, and further heated at 90°C for 5 minutes, and thereby, the Single Strand DNA Ligase was inactivated. Then, the mixture was left standing on ice for 2 minutes, and 30 µL of sterilized water was added thereto, and mixed. Furthermore, after removing unreacted adapter using Invisorb Spin PCRapid Kit (Invitek GmbH), 1 µL of Ethachinmate (Nippon Gene Co., Ltd.), 12 µL of 10 mmol/L ammonium acetate, and 125 µL of ethanol were added, respectively, and mixed; the mixture was subjected to the centrifugal separation at 18,800 x g for 10 minutes; the precipitate was washed twice with 75% ethanol and dried; and the precipitate was dissolved in 34.5 µL of sterilized water.

### 4. PCR (the 1 st time)

To an aqueous solution (34.5 µL) comprising adapter-bound reverse transcription reaction product obtained in 3, 5 µL of 10 x Reaction Buffer for HotGoldstar DNA polymerase (Eurogentec S.A.), 4 µL of 2.5 mmol/L dNTP Mixture (Nippon Gene Co., Ltd.), I µL of PCR primer which is complementary to the adapter (25 pmol/µL, 5'-CATGGTATCGACGAGACTGAG-3'), 1 µL of PCR primer which is complementary to human p16 INK4a mRNA (25 pmol/µL 5'-TATCCTCCGAACTTCTGCGG-3'), 4 µL of 25 mmol/L MgCl₂, 0.5 µL of HotGoldstar DNA Polymerase (5 U/µL: Eurogentec S.A.) were added, respectively, and mixed gently on ice; and the PCR reaction was carried out under the following condition:
95°C for 10 minutes → 95°C for 20 seconds · 55°C for 20 seconds · 72°C for 20 seconds (30 cycles) → 72°C for 2 minutes.

Subsequently, the unreacted PCR primer was removed from the obtained solution using Invisorb Spin PCRapid Kit (Invitek GmbH). Then, 1 µL of Ethachinmate (Nippon Gene Co., Ltd.), 12 µL of 10 mmol/L ammonium acetate, and 125 µL of ethanol were added respectively and mixed. Further, the mixture was subjected to centrifugal separation at 18,800 x g for 10 minutes; the precipitate was washed twice with 75% ethanol and dried; and the precipitate was dissolved in 34.5 µL of sterilized water.

### 5. PCR (the 2nd time)

To an aqueous solution (34.5 µL) obtained in 4, 5 µL of 10 x Reaction Buffer for HotGoldstar DNA polymerase (Eurogentec S.A.), 4 µL of 2.5 mmol/L dNTP Mixture (Nippon Gene Co., Ltd.), 1 µL of PCR primer which is complementary to the adapter (25 pmol/µL, 5'-CATGGTATCGACGAGACTGAG-3'), 1 µL of PCR primer which is complementary to human p16 INK4a mRNA (25 pmol/µL 5'-AGTTACACTTAGCTTCTGGG-3'), 4 µL of 25 mmol/L MgCl₂, 0.5 µL of HotGoldstar DNA Polymerase (5 U/µL: Eurogentec S.A.) were added, respectively, and mixed gently on ice; and the PCR reaction was carried out under the following condition:
95°C for 10 minutes → 95°C for 20 seconds · 55°C for 20 seconds · 72°C for 20 seconds (30 cycles) → 72°C for 2 minutes.

Subsequently, the unreacted PCR primer was removed from the obtained solution using Invisorb Spin PCRapid Kit (Invitek GmbH). Then, 1 µL of Ethachinmate (Nippon Gene Co., Ltd.), 12 µL of 10 mmol/L ammonium acetate, and 125 µL of ethanol were added, respectively, and mixed. Further, the mixture was subjected to centrifugal separation at 18,800 x g for 10 minutes; the precipitate was washed twice with 75% ethanol and dried; and the precipitate was dissolved in 4 µL of sterilized water.

### 6. Confirmation of PCR amplification product

Confirmation of chain length of the PCR amplification product was carried out using Bioanalyzer DNA 1000 Kit (Agilent Technologies). As a result, the PCR amplification product was confirmed at the position of about 160 bp. The results ares shown in Fig. 6. In addition, lane 1 in Fig. 6 shows the result of the amplification of only the PCR primer which is complementary to the adapter; lane 2 shows the result of the amplification of only the PCR primer which is complementary to the p16 INK4a mRNA; and lane 3 shows the result of the amplification using both the PCR primer which is complementary to the adapter and the PCR primer which is complementary to the p16 INK4a mRNA, respectively.

### 7. Nucleotide sequence analysis

The nucleotide sequence of the above-described PCR amplification product was analyzed using BaseStaion (Bio-Rad Laboratories, Inc.).

### (Result of nucleotide sequence analysis)

### 8. Comparison with database

Nucleotide sequence of the present cDNA was anticipated from the result of nucleotide sequence analysis of the above-described PCR amplification product, and compared with the EST database (ACCESSION No.BG717152) of human p16 INK4a which had been registered on NCBI. In consequence, it has been confirmed that the present cDNA has about 480 bp longer 5'-terminal. That is, it has been also found that, by the method of the present invention, the cDNA which is complementary to human p16 INK4a can be obtained; the cDNA which is complementary toregion from a known region to 5'-terminal containing an unknown region can be sequenced; and as a result, 5'-terminal sequence of human p16 INK4a can be determined.

The EST database of human p16 INK4a which had been registered on NCBI is shown below.

## Claims

1. A method for synthesis of single-stranded DNA containing a nucleotide sequence corresponding to template RNA, **characterized by** comprising the following steps:
1) Step 1 in which the template RNA is subjected to reverse transcription reaction;
2) Step 2 in which the solution processed in Step 1 is subjected to alkaline treatment;
3) Step 3 in which a DNA fragment of known sequence is added to 3'-terminal of reverse transcribed DNA by using a thermostable single-stranded DNA ligase after the alkaline treatment in Step 2.

2. The method of claim 1, wherein the DNA fragment addition to the reverse transcribed DNA in the step 3 is carried out at 50 to 70 °C.

3. The method of claim 1 or 2, which comprises a step in which the obtained solution is subjected to column purification or dephosphorylation treatment, prior to the step 3 in which a DNA fragment is added.

4. The method of any one of claims 1-3, which further comprises a step in which an RNA fragment of known sequence is added to 3'-terminal of the template RNA, prior to Step 1, preferably the method comprises a step in which 5'-terminal of the template RNA is dephosphorylated, prior to the step in which an RNA fragment of known sequence is added to 3'-terminal of the template RNA.

5. A method for synthesis of double-stranded DNA containing a nucleotide sequence corresponding to template RNA, **characterized by** comprising the following steps:
1) Step 1 in which the template RNA is subjected to reverse transcription reaction;
2) Step 2 in which the solution processed in the Step 1 is subjected to alkaline treatment;
3) Step 3 in which a DNA fragment of known sequence is added to 3'-terminal of reverse transcribed DNA by using a thermostable single-stranded DNA ligase after the alkaline treatment in Step 2.
4) Step 4 in which the obtained single-stranded DNA is converted to a double-stranded DNA.

6. The method of claim 5, wherein the DNA fragment addition to the reverse transcribed DNA in the step 3 is carried out at 50 to 70 °C.

7. The method of claim 5 or 6, which comprises a step in which the obtained solution is subjected to column purification or dephosphorylation treatment, prior to the step 3 in which a DNA fragment is added.

8. The method of any one of claims 5-7, which further comprises a step in which an RNA fragment of known sequence is added to 3'-terminal of the template RNA, prior to Step 1, preferably the method comprises a step in which 5'-terminal of the template RNA is dephosphorylated, prior to the step in which an RNA fragment of known sequence is added to 3'-terminal of the template RNA.

9. The method of any one of claims 5-8, wherein the double-stranded formation in Step 4 is carried out by PCR.

10. A kit for synthesis of single-stranded DNA containing a nucleotide sequence which is corresponding to template RNA, **characterized by** comprising
(i) I) an enzyme which is capable of adding RNA fragment to single-stranded RNA, II) an RNA fragment of known sequence, III) a reverse transcriptase, IV) a primer for reverse transcription reaction, V) a mixture of deoxyribonucleotide triphosphates, VII) a DNA fragment of known sequence and VIII) a thermostable single-stranded DNA ligase, and
(ii) any one or more sorts of VI) an enzyme which is capable of dephosphorylating 5'-terminal phosphate group of RNA, IX) an enzyme which is capable of dephosphorylating 5'-terminal phosphate group of DNA and X) an alkali or an aqueous solution thereof.

11. A kit for synthesis of double-stranded DNA containing a nucleotide sequence corresponding to template RNA, **characterized by** comprising I) an enzyme which is capable of adding RNA fragment to single-stranded RNA, II) an RNA fragment of known sequence, III) a reverse transcriptase, IV) a primer for reverse transcription reaction, V) a mixture of deoxyribonucleotide triphosphates, VI) an enzyme which is capable of dephosphorylating 5'-terminal phosphate group of RNA, VII) a DNA fragment of known sequence, VIII) a thermostable single-stranded DNA ligase, IX) an enzyme which is capable of dephosphorylating 5'-terminal phosphate group of DNA, and XI) a PCR primer.

12. The kit of claim 11, which further comprises X) an alkali or an aqueous solution thereof.

## Patentansprüche

1. Verfahren zur Synthese von einzelsträngiger DNA, die eine Nucleotidsequenz enthält, welche Matrizen-RNA entspricht, **dadurch gekennzeichnet, dass** es die folgenden Schritte umfasst:
1) Schritt 1, in dem die Matrizen-RNA einer reversen Transcriptionsreaktion unterzogen wird;
2) Schritt 2, in dem die in Schritt 1 verarbeitete Lösung einer alkalischen Behandlung unterzogen wird;
3) Schritt 3, in dem ein DNA-Fragment bekannter Sequenz an den 3'-Terminus der revers transcribierten DNA addiert wird, indem man nach der alkalischen Behandlung in Schritt 2 eine thermostabile einzelsträngige DNA-Ligase verwendet.

2. Verfahren gemäß Anspruch 1, wobei die Addition des DNA-Fragments an die revers transcribierte DNA in Schritt 3 bei 50 bis 70 °C durchgeführt wird.

3. Verfahren gemäß Anspruch 1 oder 2, das vor Schritt 3, in dem ein DNA-Fragment addiert wird, einen Schritt umfasst, bei dem die erhaltene Lösung einer Säulenreinigung oder Dephosphorylierungsbehandlung unterzogen wird.

4. Verfahren gemäß einem der Ansprüche 1 bis 3, das weiterhin vor Schritt 1 einen Schritt umfasst, bei dem ein RNA-Fragment bekannter Sequenz an den 3'-Terminus der Matrizen-RNA addiert wird, wobei das Verfahren vorzugsweise vor dem Schritt, bei dem ein RNA-Fragment bekannter Sequenz an den 3'-Terminus der Matrizen-RNA addiert wird, einen Schritt umfasst, bei dem der 5'-Terminus der Matrizen-RNA dephosphoryliert wird.

5. Verfahren zur Synthese von doppelsträngiger DNA, die eine Nucleotidsequenz enthält, welche Matrizen-RNA entspricht, **dadurch gekennzeichnet, dass** es die folgenden Schritte umfasst:
1) Schritt 1, in dem die Matrizen-RNA einer reversen Transcriptionsreaktion unterzogen wird;
2) Schritt 2, in dem die in Schritt 1 verarbeitete Lösung einer alkalischen Behandlung unterzogen wird;
3) Schritt 3, in dem ein DNA-Fragment bekannter Sequenz an den 3'-Terminus der revers transcribierten DNA addiert wird, indem man nach der alkalischen Behandlung in Schritt 2 eine thermostabile einzelsträngige DNA-Ligase verwendet;
4) Schritt 4, in dem die erhaltene einzelsträngige DNA in eine doppelsträngige DNA umgewandelt wird.

6. Verfahren gemäß Anspruch 5, wobei die Addition des DNA-Fragments an die revers transcribierte DNA in Schritt 3 bei 50 bis 70 °C durchgeführt wird.

7. Verfahren gemäß Anspruch 5 oder 6, das vor Schritt 3, in dem ein DNA-Fragment addiert wird, einen Schritt umfasst, bei dem die erhaltene Lösung einer Säulenreinigung oder Dephosphorylierungsbehandlung unterzogen wird.

8. Verfahren gemäß einem der Ansprüche 5 bis 7, das weiterhin vor Schritt 1 einen Schritt umfasst, bei dem ein RNA-Fragment bekannter Sequenz an den 3'-Terminus der Matrizen-RNA addiert wird, wobei das Verfahren vorzugsweise vor dem Schritt, bei dem ein RNA-Fragment bekannter Sequenz an den 3'-Terminus der Matrizen-RNA addiert wird, einen Schritt umfasst, bei dem der 5'-Terminus der Matrizen-RNA dephosphoryliert wird.

9. Verfahren gemäß einem der Ansprüche 5 bis 8, wobei die Bildung der doppelsträngigen DNA in Schritt 4 durch PCR durchgeführt wird.

10. Kit für die Synthese von einzelsträngiger DNA, die eine Nucleotidsequenz enthält, welche Matrizen-RNA entspricht, **dadurch gekennzeichnet, dass** es Folgendes umfasst:
(i) I) ein Enzym, das ein RNA-Fragment an einzelsträngige RNA addieren kann, II) ein RNA-Fragment bekannter Sequenz, III) eine Reverse Transcriptase, IV) einen Primer für die reverse Transcriptionsreaktion, V) ein Gemisch von Desoxyribonucleotidtriphosphaten, VII) ein DNA-Fragment bekannter Sequenz und VIII) eine thermostabile einzelsträngige DNA-Ligase; und
(ii) eine oder mehrere Sorten von VI) einem Enzym, das die 5'-terminale Phosphatgruppe von RNA dephosphorylieren kann, IX) einem Enzym, das die 5'-terminale Phosphatgruppe von DNA dephosphorylieren kann, und X) einer Base oder einer wässrigen Lösung davon.

11. Kit für die Synthese von doppelsträngiger DNA, die eine Nucleotidsequenz enthält, welche Matrizen-RNA entspricht, **dadurch gekennzeichnet, dass** es Folgendes umfasst: I) ein Enzym, das ein RNA-Fragment an einzelsträngige RNA addieren kann, II) ein RNA-Fragment bekannter Sequenz, III) eine Reverse Transcriptase, IV) einen Primer für die reverse Transcriptionsreaktion, V) ein Gemisch von Desoxyribonucleotidtriphosphaten, VI) ein Enzym, das die 5'-terminale Phosphatgruppe von RNA dephosphorylieren kann, VII) ein DNA-Fragment bekannter Sequenz, VIII) eine thermostabile einzelsträngige DNA-Ligase, IX) ein Enzym, das die 5'-terminale Phosphatgruppe von DNA dephosphorylieren kann, und XI) einen PCR-Primer.

12. Kit gemäß Anspruch 11, der weiterhin X) eine Base oder eine wässrige Lösung davon umfasst.

## Revendications

1. Procédé de synthèse d'un ADN simple brin contenant une séquence nucléotidique correspondant à un ARN matrice, **caractérisé en ce qu'**il comprend les étapes suivantes :
1) une étape 1 dans laquelle l'ARN matrice est soumis à une réaction de transcription inverse ;
2) une étape 2 dans laquelle la solution traitée à l'étape 1 est soumise à un traitement alcalin ;
3) une étape 3 dans laquelle un fragment d'ADN de séquence connue est ajouté à l'extrémité 3'-terminale de l'ADN soumis à une transcription inverse à l'aide d'une ADN ligase simple brin thermostable à l'issue du traitement alcalin de l'étape 2.

2. Procédé selon la revendication 1, dans lequel l'addition de fragment d'ADN à l'ADN soumis à une transcription inverse à l'étape 3 est réalisée à 50 à 70°C.

3. Procédé selon la revendication 1 ou 2, qui comprend une étape dans laquelle la solution obtenue est soumise à un traitement de purification sur colonne ou de déphosphorylation, avant l'étape 3 dans laquelle un fragment d'ADN est ajouté.

4. Procédé selon l'une quelconque des revendications 1 à 3, qui comprend en outre une étape dans laquelle un fragment d'ARN de séquence connue est ajouté à l'extrémité 3'-terminale de l'ARN matrice, avant l'étape 1, de préférence le procédé comprend une étape dans laquelle l'extrémité 5'-terminale de l'ARN matrice est déphosphorylée, avant l'étape dans laquelle un fragment d'ARN de séquence connue est ajouté à l'extrémité 3'-terminale de l'ARN matrice.

5. Procédé de synthèse d'ADN double brin contenant une séquence nucléotidique correspondant à l'ARN matrice, **caractérisé en ce qu'**il comprend les étapes suivantes :
1) une étape 1 dans laquelle l'ARN matrice est soumis à une réaction de transcription inverse ;
2) une étape 2 dans laquelle la solution traitée à l'étape 1 est soumise à un traitement alcalin ;
3) une étape 3 dans laquelle un fragment d'ADN de séquence connue est ajouté à l'extrémité 3'-terminale de l'ADN soumis à une transcription inverse à l'aide d'une ADN ligase simple brin thermostable à l'issue du traitement alcalin de l'étape 2 ;
4) une étape 4 dans laquelle l'ADN simple brin obtenu est converti en un ADN double brin.

6. Procédé selon la revendication 5, dans lequel l'addition de fragment d'ADN à l'ADN soumis à une transcription inverse à l'étape 3 est réalisée à 50 à 70°C.

7. Procédé selon la revendication 5 ou 6, qui comprend une étape dans laquelle la solution obtenue est soumise à un traitement de purification sur colonne ou de déphosphorylation, avant l'étape 3 dans laquelle un fragment d'ADN est ajouté.

8. Procédé selon l'une quelconque des revendications 5 à 7, qui comprend en outre une étape dans laquelle un fragment d'ARN de séquence connue est ajouté à l'extrémité 3'-terminale de l'ARN matrice, avant l'étape 1, de préférence le procédé comprend une étape dans laquelle l'extrémité 5'-terminale de l'ARN matrice est déphosphorylée, avant l'étape dans laquelle un fragment d'ARN de séquence connue est ajouté à l'extrémité 3'-terminale de l'ARN matrice.

9. Procédé selon l'une quelconque des revendications 5 à 8, dans lequel la formation du double brin de l'étape 4 est réalisée par PCR.

10. Coffret de synthèse d'un ADN simple brin contenant une séquence nucléotidique qui correspond à un ARN matrice, **caractérisé en ce qu'**il comprend
(i) I) une enzyme qui est capable d'ajouter un fragment d'ARN à un ARN simple brin, II) un fragment d'ARN de séquence connue, III) une transcriptase inverse, IV) une amorce pour une réaction de transcription inverse, V) un mélange de désoxyribonucléotides triphosphates, VII) un fragment d'ADN de séquence connue et VIII) une ADN ligase simple brin thermostable, et
(ii) une ou plusieurs sortes quelconques parmi VI) une enzyme qui est capable de déphosphoryler un groupe phosphate 5'-terminal d'un ARN, IX) une enzyme qui est capable de déphosphoryler un groupe phosphate 5'-terminal d'un ADN et X) un alcali ou une solution aqueuse de celui-ci.

11. Coffret de synthèse d'un ADN double brin contenant une séquence nucléotidique qui correspond à un ARN matrice, **caractérisé en ce qu'**il comprend I) une enzyme qui est capable d'ajouter un fragment d'ARN à un ARN simple brin, II) un fragment d'ARN de séquence connue, III) une transcriptase inverse, IV) une amorce pour une réaction de transcription inverse, V) un mélange de désoxyribonucléotides triphosphates, VI) une enzyme qui est capable de déphosphoryler un groupe phosphate 5'-terminal d'un ARN, VII) un fragment d'ADN de séquence connue, VIII) une ADN ligase simple brin thermostable, IX) une enzyme qui est capable de déphosphoryler un groupe phosphate 5'-terminal d'un ADN et XI) une amorce de PCR.

12. Coffret selon la revendication 11, qui comprend en outre X) un alcali ou une solution aqueuse de celui-ci.


### SEQUENCE LISTING

<110> WAKO PURE CHEMICAL INDUSTRIES, LTD.
<120> A process for synthesize of single strand DNA and double strand DNA and a kit thereof
<130> 1764
<150> JP 2007-258089
   <151> 2007-10-01
<160> 21
<170> PatentIn version 3.3
<210> 1
   <211> 19
   <212> DNA
   <213> Artificial
<220>
   <223> adaptor
<400> 1
   aagaagcugg cgucaaugu 19
<210> 2
   <211> 20
   <212> DNA
   <213> Artificial
<220>
   <223> oligonucleotide primer
<400> 2
   gacattgacg ccagcttctt 20
<210> 3
   <211> 19
   <212> DNA
   <213> Artificial
<220>
   <223> adaptor
<400> 3
   aaggctcagt ctcgggata 19
<210> 4
   <211> 18
   <212> DNA
   <213> Artificial
<220>
   <223> oligonucleotide primer
<400> 4
   gtatcccgag actgagcc 18
<210> 5
   <211> 18
   <212> DNA
   <213> Artificial
<220>
   <223> oligonucleotide primer
<400> 5
   gacattgacg ccagcttc 18
<210> 6
   <211> 18
   <212> DNA
   <213> Artificial
<220>
   <223> oligonucleotide primer
<400> 6
   gtatcccgag actgagcc 18
<210> 7
   <211> 18
   <212> DNA
   <213> Artificial
<220>
   <223> oligonucleotide primer
<400> 7
   gacattgacg ccagcttc 18
<210> 8
   <211> 20
   <212> DNA
   <213> Artificial
<220>
   <223> Olignucleotide primer
<400> 8
   tagctctgct cttgggattg 20
<210> 9
   <211> 25
   <212> DNA
   <213> Artificial
<220>
   <223> Adaptor
<400> 9
   aagcctcagt ctcgtcgata ccatg 25
<210> 10
   <211> 21
   <212> DNA
   <213> Artificial
<220>
   <223> Olignucleotide primer
<400> 10
   catggtatcg acgagactga g 21
<210> 11
   <211> 20
   <212> DNA
   <213> Artificial
<220>
   <223> Olignucleotide primer
<400> 11
   aactactcgg atcagacatc 20
<210> 12
   <211> 20
   <212> DNA
   <213> Artificial
<220>
   <223> Olignucleotide primer
<400> 12
   agtaatgttc cctccctatc 20
<210> 13
   <211> 178
   <212> DNA
   <213> Mouse
<400> 13
   atatctctga aaacctccag cgtattctgg tagtccagag tggatgggga ttcagggagc 60
   ctgggtttgc acacttggaa aatgtacctc aagatcctta atataatata gtgcttaata 120
   cagtgctgtc tactatgtct ttcctctcta gttacagttc aaaggacgaa taatttaa 178
<210> 14
   <211> 996
   <212> DNA
   <213> Mouse
<400> 14
   atatctctga aaacctccag cgtattctgg tagtccagag tggatgggga ttcagggagc 60
   ctgggtttgc acacttggaa aatgtacctc aagatcctta atataatata gtgcttaata 120
   cagtgctgtc tactatgtct ttcctctcta gttacagttc aaaggacgaa taatttaaga 180
   tagggaggga acattactat tttagggaca tgctttctta ttttatactt aaagaaattg 240
   aagtataaca ttccagaaag actaggttaa caaaaaaaaa aaaaagtgag aattctggtt 300
   gtcttttttt ttgccaacca cttttttagt aattaccaca caatcccagt tcggcttaaa 360
   ggcaagtgct gtgtgtctat gctcccggcg atgttctaca ggagtttgag taccaggaaa 420
   tcttagggaa tacactgtaa gcctgtgtgt aagaagaatt ccaaggcggg actaggagcc 480
   tttatggggt agagcctcct gactgtggat gtctgatccg agtagttaac agcggagctt     540
   cgtacatagg gcttctttct tgggtcctgc cctcactctg gccgtgatcc ctctactttt     600
   tcttctgact tttcaggtga tgatgatggg caacgttcac gtagcagctc ttctgctcaa     660
   ctacggtgca gattcgaact gcgaggaccc cactaccttc tcccgcccgg tgcacgacgc     720
   agcgcgggaa ggcttcctgg acacgctggt ggtgctgcac gggtcagggg ctcggctgga     780
   tgtgcgcgat gcctggggtc gcctgccgct cgacttggcc caagagcggg gacatcaaga     840
   catcgtgcga tatttgcgtt ccgctgggtg ctctttgtgt tccgctgggt ggtctttgtg     900
   taccgctggg aacgtcgccc agaccgacgg gcatagcttc agctcaagca cgcccagggc     960
   cctggaactt cgcggccaat cccaagagca gagcta                               996
<210> 15
   <211> 675
   <212> DNA
   <213> Mouse
<220>
   <221> mRNA
   <222> (596)..(596)
   <223> n is a, c, g, or t
<220>
   <221> mRNA
   <222> (645)..(645)
   <223> n is a, c, g, or t
<220>
   <221> mRNA
   <222> (667)..(667)
   <223> n is a, c, g, or t
<400> 15
   tgggcgattg ggcgggcact gaatctccgc gaggaaagcg aactcgagga gagccatctg      60
   gagcagcatg gagtccgctg cagacagact ggccagggcg gcggcccagg gccgtgtgca     120
   tgacgtgcgg gcactgctgg aagccggggt ttcgcccaac gccccgaact ctttcggtcg     180
   taccccgatt caggtgatga tgatgggcaa cgttcacgta gcagctcttc tgctcaacta     240
   cggtgcagat tcgaactgcg aggaccccac taccttctcc cgcccggtgc acgacgcagc     300
   gcgggaaggc ttcctggaca cgctggtggt gctgcacggg tcaggggctc ggctggatgt     360
   gcgcgatgcc tggggtcgcc tgccgctcga cttggcccaa gagcggggac atcaagacat     420
   cgtgcgatat ttgcgttccg ctgggtgctc tttgtgttcc gctgggtggt ctttgtgtac     480
   cgctgggaac gtcgcccaga ccgacgggca tagcttcagc tcaagcacgc ccagggccct     540
   ggaacttcgc ggccaatccc aagagcagag ctaaatccgg cctcagcccg cctttntctt     600
   cttagcttca cttctagcga tgctagcgtg tctagcatgt ggctntaaaa aatacataat     660
   aatgctnttt ttgca                                                      675
<210> 16
   <211> 20
   <212> DNA
   <213> Artificial
<220>
   <223> Olignucleotide primer
<400> 16
   ttctcagagc ctctctggtt 20
<210> 17
   <211> 20
   <212> DNA
   <213> Artificial
<220>
   <223> Olignucleotide primer
<400> 17
   tatcctccga acttctgcgg 20
<210> 18
   <211> 20
   <212> DNA
   <213> Artificial
<220>
   <223> Olignucleotide primer
<400> 18
   agttacactt agcttctggg 20
<210> 19
   <211> 108
   <212> DNA
   <213> human
<400> 19
   tcaaagatca taacttcata ttgtaccaca taaatatata caactgtact atcccaatat 60
   ataattttaa aactaatata atgaaaaaga aattgaagtt caacattc 108
<210> 20
   <211> 947
   <212> DNA
   <213> human
<400> 20
   tcaaagatca taacttcata ttgtaccaca taaatatata caactgtact atcccaatat 60
   ataattttaa aactaatata atgaaaaaga aattgaagtt caacattccc agaagctaag 120
   tgtaacttaa aagttttgtg agaatttgtt ttaacaaaca aacaagtttt ctctttttaa 180
   caattaccac attctgcgct tggatataca gcagtgaaca aaaaaaaaaa aaaaaatctc 240
   caggcctaac ataatttcag gaagaaattt cagtagttgt atctcagggg aaatacagga 300
   agttagcctg gagtaaaagt cagtctgtcc ctgccccttt gctattttgc ccgtgcctca 360
   cagtgctctc tgcctgtgac gacagctccg cagaagttcg gaggatataa tggaattcat 420
   tgtgtactga agaatggata gagaactcaa gaaggaaatt ggaaactgga agcaaatgta 480
   ggggtaatta gacacctggg gcttgtgtgg gggtctgctt ggcggcgagg gggctctaca 540
   caagcttcct ttccgtcatg ccggccccca ccctggctct gaccattctg ttctctctgg 600
   caggtcatga tgatgggcag cgcccgagtg gcggagctgc tgctgctcca cggcgcggag 660
   cccaactgcg ccgaccccgc cactctcacc cgacccgtgc acgacgctgc ccgggagggc 720
   ttcctggaca cgctggtggt gctgcaccgg gccggggcgc ggctggacgt gcgcgatgcc 780
   tggggccgtc tgcccgtgga cctggctgag gagctgggcc atcgcgatgt cgcacggtac 840
   ctgcgcgcgg ctgcgggggg caccagaggc agtaaccatg cccgcataga tgccgcggaa 900
   ggtccctcag acatccccga ttgaaagaac cagagaggct ctgagaa 947
<210> 21
   <211> 462
   <212> DNA
   <213> human
<400> 21
   aattagacac ctggggcttg tgtgggggtc tgcttggcgg cgagggggct ctacacaagc      60
   ttcctttccg tcatgccggc ccccaccctg gctctgacca ttctgttctc tctggcaggt     120
   catgatgatg ggcagcgccc gagtggcgga gctgctgctg ctccacggcg cggagcccaa     180
   ctgcgccgac cccgccactc tcacccgacc cgtgcacgac gctgcccggg agggcttcct     240
   ggacacgctg gtggtgctgc accgggccgg ggcgcggctg gacgtgcgcg atgcctgggg     300
   ccgtctgccc gtggacctgg ctgaggagct gggccatcgc gatgtcgcac ggtacctgcg     360
   cgcggctgcg gggggcacca gaggcagtaa ccatgcccgc atagatgccg cggaaggtcc     420
   ctcagacatc cccgattgaa agaaccagag aggctctgag aa                        462